(19) Europäisches Patentamt
European Patent Office
Office européen des brevets

(11) Numéro de publication: **0 072 743**
**A1**

(12) **DEMANDE DE BREVET EUROPEEN**

(21) Numéro de dépôt: 82401502.8

(22) Date de dépôt: 06.08.82

(51) Int. Cl.³: C 07 D 339/04
C 07 D 409/02, A 61 K 31/385

(30) Priorité: 11.08.81 FR 8115528
17.06.82 FR 8210616

(43) Date de publication de la demande:
23.02.83 Bulletin 83/8

(84) Etats contractants désignés:
AT BE CH DE FR GB IT LI LU NL SE

(71) Demandeur: RHONE-POULENC SANTE
Les Miroirs 18 Avenue d'Alsace
F-92400 Courbevoie Cedex(FR)

(72) Inventeur: Cotrel, Claude
17 A, avenue du Docteur Arnold Netter
F-75012 Paris(FR)

(72) Inventeur: Farge, Daniel
30, rue des Pins Sylvestres
F-94320 Thiais(FR)

(72) Inventeur: Taurand, Gérard
35, rue de la Clairière
F-94370 Sucy-En-Brie(FR)

(74) Mandataire: Le Goff, Yves et al,
RHONE-POULENC RECHERCHES Brevets Pharma 25,
Quai Paul Doumer
F-92408 Courbevoie(FR)

(54) **Nouveaux dérivés du dithiole-1,2 ylidène-3 ammonium, leur préparation et les médicaments qui les contiennent.**

(57) Nouveaux dérivés de formule (I) dans laquelle $X^{\ominus}$ est un anion, R est alcoyle (1 à 7 C) [non substitué ou substitué par hydroxy, carboxy, alcoyloxycarbonyle, cyano, dialcoylamino, alcoylcarbonyle, benzoyle non substitué ou substitué par un ou plusieurs halogènes, ou par alcoyle (éventuellement substitué par un ou plusieurs halogènes), alcoyloxy, hydroxy, amino, alcoylamino, dialcoylamino, cyano ou nitro, soit un radical thénoyle (non substitué ou substitué par un ou plusieurs halogènes ou par alcoyle, cyano ou nitro), soit un radical pyridinecarbonyle, dialcoylcarbamoyle (dont les parties alcoyle peuvent former un hétérocycle à 5 ou 6 chaînons), pyridyle], dialcoylcarbamoyle (dont les parties alcoyle peuvent former un hétérocycle à 5 ou 6 chaînons), alcényle ou alcynyle (2 à 4 C), alcoyloxycarbonyle, un cycle oxo-2 tétrahydrofuryle-3 ou oxo-2 tétrahydropyrannyle-3 et
- soit $R_1$ et $R_2$ sont phényle, cycloalcoyle, alcoyle ou phénylalcoyle ou forment ensemble un hétérocycle à 5 à 7 chaînons
- soit $R_1$ est phényle, éventuellement substitué par alcoyle (pouvant être substitué par un ou plusieurs halogènes), alcoyloxy, hydroxy, amino, alcoylamino, dialcoylamino, cyano ou nitro ou bien est cycloalcoyle, alcoyle ou phénylalcoyle et $R_2$ est un atome d'hydrogène.

Les nouveaux produits de formule (I) sont utiles comme anti-ulcères cytoprotecteurs.

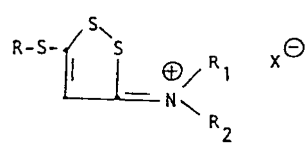

(1)

EP 0 072 743 A1

La présente invention concerne de nouveaux dérivés du dithiole-1,2 ylidène-3 ammonium de formule générale :

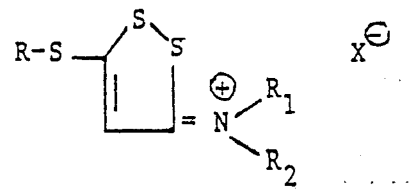

(I)

dans laquelle $X^{\ominus}$ représente un anion, R représente un radical alcoyle contenant 1 à 7 atomes de carbone en chaîne droite ou ramifiée [non substitué ou substitué soit par un radical hydroxy, carboxy, alcoyloxycarbonyle, cyano, dialcoylamino, alcoylcarbonyle, benzoyle dont le cycle phényle est ~~non substitué ou~~ substitué par un ou plusieurs atomes d'halogène, ou radicaux choisis parmi alcoyle (éventuellement substitué par un ou plusieurs atomes d'halogène), alcoyloxy, hydroxy, amino, alcoylamino, dialcoylamino, cyano ou nitro, soit par un radical théncyle dont le cycle thiényle est substitué par un ou plusieurs atomes d'halogène ou radicaux choisis parmi alcoyle, cyano ou nitro, pyridylcarbonyle, carbamoyle, dialcoylcarbamoyle (dont les parties alcoyle peuvent former ensemble avec l'atome d'azote auquel elles sont liées un hétérocycle à 5 ou 6 chaînons contenant éventuellement un autre hétéroatome choisi parmi l'oxygène, le soufre ou un atome d'azote substitué par un radical alcoyle ou alcoylcarbonyle), pyridyle], dialcoylcarbamoyle (dont les parties alcoyle peuvent former ensemble avec l'atome d'azote auquel elles sont liées un hétérocycle à 5 ou 6 chaînons contenant éventuellement un autre hétéroatome choisi parmi l'oxygène, le soufre ou un atome d'azote substitué par un radical alcoyle ou alcoylcarbonyle), alcényle contenant 2 à 4 atomes de carbone, alcynyle contenant 2 à 4 atomes de carbone, alcoyloxycarbonyle ou bien représente un cycle oxo-2 tétrahydrofuryle-3 ou oxo-2 tétrahydropyrannyle-3 et soit $R_1$ et $R_2$, identiques ou différents, représentent un radical phényle, cycloalcoyle contenant 3 à 7 atomes de carbone, alcoyle ou phénylalcoyle ou bien forment ensemble avec l'atome d'azote auquel ils sont liés un hétérocycle à 5 à 7 chaînons pouvant contenir éventuellement un autre hétéroatome choisi parmi l'oxygène, le soufre ou un atome d'azote substitué par un radical alcoyle,

soit $R_1$ représente un radical phényle non substitué ou substitué par un ou plusieurs atomes d'halogène ou radicaux choisis parmi alcoyle (éventuellement substitué par un ou plusieurs atomes d'halogène), alcoyloxy, hydroxy, amino, alcoylamino, dialcoylamino, cyano ou nitro, ou bien représente un radical cycloalcoyle contenant 3 à 7 atomes de carbone, alcoyle ou phénylalcoyle et $R_2$ représente un atome d'hydrogène, ainsi que les bases correspondantes lorsque $R_2$ est hydrogène, étant entendu que, sauf mention spéciale, les radicaux et portions alcoyle cités ci-dessus ou qui seront cités par la suite contiennent 1 à 4 atomes de carbone et sont en chaîne droite ou ramifiée.

Selon l'invention, les produits de formule générale (I) peuvent être préparés par action d'un dérivé de formule générale :

$$R - X_1 \qquad (II)$$

dans laquelle R est défini comme précédemment et $X_1$ représente un atome d'halogène tel que chlore, brome ou iode ou un reste d'ester réactif tel qu'un radical mésyloxy ou tosyloxy, sur une dithiole-1,2 thione-3 de formule générale :

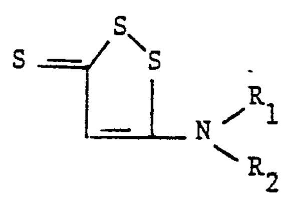

(III)

dans laquelle $R_1$ et $R_2$ sont définis comme précédemment puis on isole le produit obtenu et le transforme éventuellement en un autre sel ou en base correspondante si elle existe, c'est-à-dire si dans les produits de formule générale (I) $R_2$ représente un atome d'hydrogène.

On peut opérer dans un solvant organique inerte à une température comprise entre 40°C et la température de reflux du mélange réactionnel.

Pour accroître la réactivité du dérivé $RX_1$ lorsque $X_1$ est différent d'un atome d'iode, il est souvent avantageux d'ajouter de l'iodure de sodium au mélange réactionnel en quantité au moins stoechiométrique ; le sel isolé dans ce cas est généralement l'iodure.

Les dithiole-1,2 thione-3 de formule générale (III) peuvent être préparées par cyclisation d'un ester propionique de formule générale :

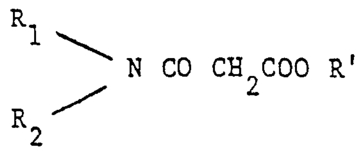

(IV)

dans laquelle $R_1$ et $R_2$ sont définis comme précédemment et R' représente un radical alcoyle, au moyen d'un réactif de thionation tel que $P_4S_{10}$ ou le réactif de PEDERSEN-LAWESSON [bis(méthoxy-4 phényl)-2,4 dithioxo-2,4 dithia-1,3 diphospha-2,4 étanne]. On opère dans des conditions analogues à celles décrites par B.S. PEDERSEN et S.O. LAWESSON, Tetrahedron 35, 2433 (1979).

Les esters propioniques de formule générale (IV) peuvent être préparés par action d'une amine de formule générale :

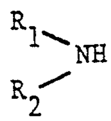

(V)

sur un chloroformylacétate d'alcoyle ou selon la méthode de F.D. CHATTAWAY et Coll., J. Chem. Soc. 97, 939 (1910).

Les nouveaux produits de formule générale (I) dans lesquels $R_2$ représente un atome d'hydrogène peuvent être transformés en bases correspondantes par action d'un bicarbonate alcalin, de la soude ou de la potasse en milieu aqueux. Si la base précipite dans le milieu, elle est isolée par filtration ; sinon elle est extraite à l'aide d'un solvant organique. On peut obtenir directement la base sans isoler le sel intermédiaire en opérant la condensation de (II) et (III) en présence d'un accepteur de proton comme la triéthylamine.

Les bases ainsi obtenues peuvent être transformées en sels de formule générale (I) par addition d'acides, dans des solvants appropriés. Comme solvants, on utilise par exemple des alcools, des

cétones, des éthers ou des solvants chlorés. Le sel formé précipite, éventuellement après concentration de sa solution ; il est séparé par filtration ou décantation.

Selon l'invention, les nouveaux produits de formule générale (I) dans laquelle $R_1$ représente un radical phényle, cycloalcoyle, alcoyle ou phénylalcoyle et $R_2$ représente un radical cycloalcoyle, alcoyle ou phénylalcoyle peuvent être également préparés par action d'un dérivé de formule générale :

$$R'_2 X_2 \qquad \text{(VI)}$$

dans laquelle $R'_2$ représente un radical cycloalcoyle, alcoyle ou phénylalcoyle et $X_2$ représente un atome d'halogène ou un reste d'ester réactif tel qu'un radical mésyloxy ou tosyloxy, sur un produit de formule générale (I) dans laquelle $R_1$ représente un radical phényle, cycloalcoyle, alcoyle ou phénylalcoyle et $R_2$ représente un atome d'hydrogène.

On peut opérer dans un solvant organique inerte habituellement utilisé pour quaterniser une amine.

Les produits de formule générale (I) peuvent être transformés en un autre sel par toute méthode de transsalification connue en soi et compatible avec la nature de l'anion initial.

Les nouveaux produits de formule générale (I) peuvent être purifiés par les méthodes habituelles, notamment la cristallisation et la chromatographie.

On connaît des dérivés de dithiole-1,3 ylidène-3 amine par la publication de F. BOBERG et Coll., J. Prakt. Chem. 315, 970 (1973), par la publication de G. CAILLAUD et Y. MOLLIER, Bull. Soc. Chim. France 72, 147 (1972) et par le brevet FR 2 011 918. Les produits décrits dans ce brevet sont présentés comme antifongiques agricoles. Aucun des trois documents mentionnés ci-dessus ne signale des propriétés pharmacologiques pour les produits décrits.

Les nouveaux produits de formule générale (I) et leurs bases, lorsqu'elles existent, présentent d'intéressantes propriétés pharmacologiques les rendant utiles comme anti-ulcères cytoprotecteurs.

Ils sont en particulier actifs dans le test de l'ulcère à l'éthanol à des doses comprises entre 1 et 100 mg/kg par voie orale chez le rat selon la technique de A. ROBERT, Gastroenterology, 77, 433 (1979.

Sont particulièrement intéressants les produits de formule générale (I) dans laquelle R représente un radical alcényle contenant 2 à 4 atomes de carbone, alcoyle contenant 1 à 7 atomes de carbone en chaîne droite ou ramifiée [non substitué ou substitué par un radical cyano, dialcoylamino, carbamoyle, alcoylcarbonyle, thénoyle, benzoyle dont le cycle phényle est non substitué ou substitué (par un ou plusieurs atomes d'halogène, ou radicaux choisis parmi alcoyle, alcoyloxy, hydroxy ou cyano)], et $R_1$ et $R_2$ forment ensemble un cycle pyrrolidine ou morpholine.

Sont plus particulièrement intéressants, les produits de formule générale (I) dans laquelle R représente le radical méthyle ou éthyle non substitué ou substitué par un radical benzoyle dont le cycle phényle est non substitué ou substitué par un ou plusieurs atomes d'halogène ou radicaux choisis parmi alcoyle, alcoyloxy, hydroxy ou cyano et $R_1$ et $R_2$ forment ensemble le cycle morpholine.

Sont plus spécialement intéressants les produits suivants:

- le chlorure de N-[(chloro-4 phénacylthio)-5 dithiole-1,2 ylidène-3] morpholinium
- le chlorure de N-[(méthoxy-3 phénacylthio)-5 dithiole-1,2 ylidène-3] morpholinium
- le chlorure de N-[fluoro-4 phénacylthio)-5 dithiole-1,2 ylidène-3] morpholinium
- le chlorure de N-[(dichloro-2,4 phénacylthio)-5 dithiole-1,2 ylidène-3] morpholinium
- l'iodure de N-[(chloro-2 phénacylthio)-5 dithiole-1,2 ylidène-3] morpholinium
- le chlorure de N-[(hydroxy-4 phénacylthio)-5 dithiole-1,2 ylidène-3] morpholinium
- l'iodure de N-[(méthoxy-4 phénacylthio)-5 dithiole-1,2 ylidène-3 morpholinium
- le chlorure de N-[(méthyl-4 phénacylthio)-5 dithiole-1,2 ylidène-3] morpholinium

- le chlorure de N-(cyano-4 phénacylthio)-5 dithiole-1,2 ylidène-3] morpholinium
- le chlorure de N-[(phénacylthio)-5 dithiole-1,2 ylidène-3] morpholinium

Pour l'emploi médicinal, il est fait usage des nouveaux composés à l'état de bases lorsqu'elles existent ou à l'état de sels pharmaceutiquement acceptables obtenus soit directement par condensation des produits de formules générales (II) et (III), soit par salification des bases libres lorsqu'elles existent, soit encore par transsalification, comme il a été dit plus haut.

Comme anions pouvant conduire à des sels pharmaceutiquement acceptables on peut citer des anions minéraux (tels que les chlorures, bromures,iodures,sulfates, nitrates, phosphates) ou organiques (tels que les acétates, propionates, succinates, benzoates, fumarates, maléates, théophylline-acétates, salicylates, phénolphtalinates, méthylène-bis-β-oxynaphtoates) ou des dérivés de substitution de ces composés.

Les exemples suivants, donnés à titre non limitatif, montrent comment l'invention peut être mise en pratique.

EXEMPLE 1

A une suspension de 17,7 g de diméthylamino-5 dithiole-1,2 thione-3 dans 350 cm3 d'acétone, on ajoute 21,3 g d'iodure de méthyle et chauffe au reflux pendant 1 heure. Après refroidissement le produit insoluble formé est séparé par filtration et lavé 2 fois avec 20 cm3 d'acétone. Après deux recristallisations successives de ce produit brut dans l'eau on obtient 14,2 g d'iodure de N-(méthylthio-5 dithiole-1,2 ylidène-3) diméthylammonium fondant à 226°C.

La diméthylamino-5 dithiole-1,2 thione-3 peut être préparée par action de 51 g de diméthylamino-3 oxo-3 propionate d'éthyle sur 107,5 g de pentasulfure de phosphore dans 640 cm3 de pyridine au reflux pendant 1 heure. Après refroidissement à une température voisine de 20°C le mélange réactionnel est versé dans 6,4 litres d'eau et le produit insoluble est extrait avec 1 litre de chlorure de méthylène.

La phase aqueuse est ensuite séparée par décantation et lavée 2 fois avec 500 cm3 de chlorure de méthylène. Les phases chlorométhyléniques sont réunies, séchées sur sulfate de sodium puis concentrées à sec sous pression réduite (20 mm de mercure ; 2,7 kPa) à 40°C. Le résidu est dissous dans 1 litre de chlorure de méthylène et la solution ainsi obtenue est versée sur 1,1 kg de gel de silice contenu dans une colonne de 5,9 cm de diamètre. On élue avec 8 litres de chlorure de méthylène ; cet éluat est éliminé. On élue ensuite avec 4 litres de chlorure de méthylène et l'éluat correspondant est concentré à sec sous pression réduite (20 mm de mercure ; 2,7 kPa) à 40°C. Le résidu obtenu est repris par 20 cm3 d'acétate d'éthyle et le produit insoluble est séparé par filtration puis lavé 2 fois avec 10 cm3 d'acétate d'éthyle. Après séchage on obtient 8,6 g de diméthylamino-5 dithiole-1,2 thione-3 fondant à 193°C.

Le diméthylamino-3 oxo-3 propionate d'éthyle peut être préparé selon la méthode décrite par A. ERMILI et coll., J. Org. Chem. 30, 339 (1965).

EXEMPLE 2

A une solution de 1,45 g de diéthylamino-5 dithiole-1,2 thione-3 dans 25 cm3 d'acétone au reflux, on ajoute une solution de 1,42 g d'iodure de méthyle dans 4 cm3 d'acétone et on maintient le mélange réactionnel au reflux pendant 1 heure 30 minutes. Après refroidissement à une température voisine de 20°C, le produit insoluble formé est séparé par filtration puis lavé avec 3 fois 5 cm3 d'acétone puis avec 2 fois 5 cm3 d'oxyde d'isopropyle.

Par recristallisation du produit ainsi obtenu dans 8,7 cm3 d'éthanol, on obtient 2 g d'iodure de N-(méthylthio-5 dithiole-1,2 ylidène-3) diéthylammonium fondant à 159°C.

La diéthylamino-5 dithiole-1,2 thione-3 peut être préparée par action de 12,4 g de pentasulfure de phosphore sur 7 g de diéthylamino-3 oxo-3 propionate d'éthyle dans 92 cm3 de pyridine au reflux pendant 1 heure. Le mélange réactionnel est refroidi à une température voisine de 20°C, additionné de 925 cm3 d'eau et extrait successivement par 105 cm3 et 2 fois 75 cm3 de chlorure de méthylène. Les phases

organiques sont réunies, lavées avec 3 fois 50 cm3 d'ammoniaque 4N puis avec 50 cm3 d'eau distillée, séchées sur sulfate de magnésium, filtrées et concentrées à sec sous pression réduite (20 mm de mercure ; 2,7 kPa) à 40°C. Le résidu obtenu est dissous dans 10 cm3 de chlorure de méthylène et la solution obtenue est versée sur 110 g de gel de silice contenus dans une colonne de 3 cm de diamètre. On élue d'abord avec 650 cm3 de chlorure de méthylène ; l'éluat correspondant est éliminé. On élue ensuite avec 1500 cm3 de chlorure de méthylène ; l'éluat correspondant est concentré à sec sous pression réduite (20 mm de mercure ; 2,7 kPa) à 40°C. Le résidu obtenu est repris avec 5 cm3 d'oxyde de diéthyle et le produit insoluble est séparé par filtration puis lavé par 3 fois 3 cm3 d'éther éthylique. Après séchage, on obtient 1,5 g de diéthylamino-5 dithiole-1,2 thione-3 fondant à 93°C.

Le diéthylamino-3 oxo-3 propionate d'éthyle peut être préparé par action de 7,3 g de diéthylamine sur 15 g de chloroformylacétate d'éthyle en présence de 10,1 g de triéthylamine dans 100 cm3 de chlorure de méthylène à une température voisine de 20°C pendant 2 heures. Après hydrolyse par 100 cm3 d'eau distillée, la phase organique est lavée successivement avec 100 cm3 d'une solution aqueuse d'acide chlorhydrique 1N, 4 fois 35 cm3 d'eau distillée, avec 178 cm3 d'une solution aqueuse de bicarbonate de sodium à 0,4 % enfin avec 75 cm3 d'eau distillée, puis est séchée sur sulfate de magnésium, filtrée et concentrée à sec sous pression réduite (20 mm de mercure ; 2,7 kPa) à 40°C. On obtient ainsi 14,6 g de diéthylamino-3 oxo-3 propionate d'éthyle sous forme d'une huile orange. [Rf = 0,64 ; chromatographie sur couche mince de gel de silice ; solvant : acétate d'éthyle].

EXEMPLE 3

A une solution de 2,5 g de (N-méthylbutylamino)-5 dithiole-1,2 thione-3 dans 38 cm3 d'acétone au reflux, on ajoute une solution de 2,35 g d'iodure de méthyle dans 7 cm3 d'acétone et on maintient le reflux pendant 1 heure 30 minutes. Après refroidissement à une température voisine de 20°C, le produit insoluble formé est séparé par filtration et lavé avec 3 fois 5 cm3 d'acétone puis avec 2 fois 5 cm3 d'éther éthylique.

Par recristallisation du produit ainsi obtenu dans 19 cm3 d'éthanol, on obtient 2,95 g d'iodure de N-méthyl N-(méthylthio-5 dithiole-1,2 ylidène-3) butylammonium fondant à 135°C.

La (N-méthylbutylamino)-5 dithiole-1,2 thione-3 peut être préparée par action de 114 g de pentasulfure de phosphore sur 69,4 g de (N-méthylbutylamino)-3 oxo-3 propionate d'éthyle dans 850 cm3 de pyridine au reflux pendant 1 heure. Le mélange réactionnel est ensuite refroidi à une température voisine de 20°C puis hydrolysé par 8,5 litres d'eau distillée et extrait avec 980 puis 2 fois 700 cm3 de chlorure de méthylène. Les phases organiques sont réunies, lavées avec 3 fois 460 cm3 d'ammoniaque 4N puis avec 460 cm3 d'eau distillée, séchées sur sulfate de magnésium en présence de noir décolorant, filtrées et concentrées à sec sous pression réduite (20 mm de mercure ; 2,7 kPa) à 40°C. Le résidu est dissous dans 100 cm3 de chlorure de méthylène et la solution obtenue est versée sur 1000 g de gel de silice contenus dans une colonne de 6 cm de diamètre. On élue d'abord avec 2300 cm3 de chlorure de méthylène ; l'éluat correspondant est éliminé. On élue ensuite 13,6 litres de chlorure de méthylène ; l'éluat correspondant est concentré à sec sous pression réduite (20 mm de mercure ; 2,7 kPa) à 40°C. Le résidu ainsi obtenu est dissous dans 50 cm3 de chlorure de méthylène et la solution est versée sur 400 g de gel de silice contenus dans une colonne de 4 cm de diamètre. On élue avec 750 cm3 de chlorure

de méthylène ; l'éluat correspondant est éliminé. On élue ensuite avec 1600 cm3 de chlorure de méthylène ; l'éluat correspondant est concentré à sec sous pression réduite (20 mm de mercure ; 2,7 kPa) à 40°C. On obtient ainsi 12,7 g de (N-méthylbutylamino)-5 dithiole-1,2 thione-3 sous forme d'une huile orange.

[Rf = 0,78 ; chromatographie sur couche mince de gel de silice ; solvant : acétate d'éthyle].

Le (N-méthylbutylamino)-3 oxo-3 propionate d'éthyle peut être préparé par action de 34,7 g de N-méthylbutylamine sur 60 g de chloroformylacétate d'éthyle en présence de 40,3 g de triéthylamine dans 400 cm3 de chlorure de méthylène à une température voisine de 20°C pendant 2 heures. Après hydrolyse par 400 cm3 d'eau distillée, on lave la phase organique successivement avec 325 cm3 d'une solution aqueuse d'acide chlorhydrique 1N, 300 puis 2 fois 145 cm3 d'eau distillée, 310 cm3 d'une solution aqueuse à 0,44 % de bicarbonate de sodium et 300 cm3 d'eau distillée, sèche sur sulfate de magnésium, filtre et concentre à sec sous pression réduite (20 mm de mercure ; 2,7 kPa) à 40°C. On obtient ainsi 69,4 g de (N-méthylbutylamino)-3 oxo-3 propionate d'éthyle sous forme d'une huile orange.

[Rf = 0,52 ; chromatographie sur couche mince de gel de silice ; solvant : acétate d'éthyle].

EXEMPLE 4

A une solution de 3,5 g de (N-méthyl phénéthylamino)-5 dithiole-1,2 thione-3 dans 45 cm3 d'acétone au reflux, on ajoute une solution de 5,55 g d'iodure de méthyle dans 10 cm3 d'acétone et on maintient le mélange réactionnel au reflux pendant 1 heure 30 minutes. Après refroidissement à une température voisine de 20°C, le produit insoluble formé est séparé par filtration et lavé avec 10 cm3 d'acétone.

Par recristallisation du produit ainsi obtenu dans 600 cm3 d'éthanol, on obtient 4,6 g d'iodure de N-méthyl N-(méthylthio-5 dithiole-1,2 ylidène-3) phénéthylammonium fondant à 186-187°C.

La (N-méthyl phénéthylamino)-5 dithiole-1,2 thione-3 peut être préparée par action de 38,4 g de pentasulfure de phosphore sur 28,7 g de (N-méthyl phénéthylamino)-3 oxo-3 propionate d'éthyle dans 290 cm3 de pyridine au reflux pendant 1 heure. Le mélange réactionnel est refroidi à une température voisine de 20°C puis hydrolysé par 3500 cm3 d'eau distillée et extrait par 6 fois 500 cm3 de chlorure de méthylène. Les phases organiques sont réunies, lavées avec 4 fois 400 cm3 d'eau distillée, séchées sur sulfate de magnésium et concentrées à sec sous pression réduite (20 mm de mercure ; 2,7 kPa) à 40°C. Le résidu est dissous dans 100 cm3 de chlorure de méthylène et la solution est versée sur 1200 g de gel de silice contenus dans une colonne de 6 cm de diamètre. On élue d'abord avec 3700 cm3 de chlorure de méthylène ; l'éluat correspondant est éliminé. On élue ensuite avec 5700 cm3 de chlorure de méthylène ; l'éluat correspondant est concentré à sec sous pression réduite (20 mm de mercure ; 2,7 kPa) à 40°C. Le résidu est recristallisé dans 25 cm3 d'acétate d'éthyle. On obtient ainsi 3,7 g de (N-méthyl phénéthylamino)-5 dithiole-1,2 thione-3 fondant à 96-98°C.

Le (N-méthyl phénéthylamino)-3 oxo-3 propionate d'éthyle peut être préparé par action de 15,6 g de N-méthylphénéthylamine sur 18,7 g de chloroformylacétate d'éthyle en présence de 12,6 g de triéthylamine dans 125 cm3 de chlorure de méthylène à une température voisine de 20°C pendant 3 heures. Après hydrolyse par 100 cm3 d'eau distillée, la phase organique est lavée avec 100 cm3 d'une solution aqueuse d'acide chlorhydrique 1N puis avec 2 fois 100 cm3 d'eau distillée, séchée sur sulfate de magnésium, filtrée et concentrée à sec sous pression réduite (20 mm de mercure ; 2,7 kPa) à 40°C. On obtient ainsi 28,7 g de (N-méthyl phénéthylamino)-3 oxo-3 propionate

d'éthyle sous forme d'une huile orangée.

[Rf = 0,64 ; chromatographie sur couche mince de gel de silice ; solvant : acétate d'éthyle].

EXEMPLE 5

A une solution de 3,6 g de (N-méthylbenzylamino)-5 dithiole-1,2 thione-3 dans 45 cm3 d'acétone au reflux, on ajoute une solution de 6,3 g d'iodure de méthyle dans 10 cm3 d'acétone et on maintient le reflux pendant 1 heure. Après refroidissement à une température voisine de 20°C, le produit insoluble formé est séparé par filtration et lavé avec 15 cm3 d'acétone.

Par recristallisation du produit ainsi obtenu dans 200 cm3 d'éthanol, on obtient 3,7 g d'iodure de N-méthyl N-(méthylthio-5 dithiole-1,2 ylidène-3) benzylammonium fondant à 171-173°C.

La (N-méthylbenzylamino)-5 dithiole-1,2 thione-3 peut être préparée par action de 93 g de pentasulfure de phosphore sur 66 g de (N-méthylbenzylamino)-3 oxo-3 propionate d'éthyle dans 650 cm3 de pyridine au reflux pendant 1 heure. Après refroidissement à une température voisine de 20°C le mélange réactionnel est hydrolysé par 6 litres d'eau distillée et extrait par 5 fois 500 cm3 de chlorure de méthylène. Les phases organiques sont réunies, lavées successivement avec 2 fois 500 cm3 d'eau distillée, 1000 cm3 d'ammoniaque 2N et 2 fois 500 cm3 d'eau distillée, séchées sur sulfate de magnésium, filtrées et concentrées à sec sous pression réduite (20 mm de mercure ; 2,7 kPa) à 40°C. Le résidu est dissous dans 100 cm3 de chlorure de méthylène et la solution est versée sur 1000 g de gel de silice contenus dans une colonne de 5,5 cm de diamètre. On élue d'abord avec 6 litres de chlorure de méthylène ; l'éluat correspondant est éliminé. On élue ensuite avec 10 litres de chlorure de méthylène ; l'éluat correspondant est concentré à sec sous pression réduite (20 mm de mercure ; 2,7 kPa) à 40°C. Le résidu est repris par 85 cm3 d'acétate d'éthyle et le produit insoluble est séparé par filtration. Après séchage on obtient 13,3 g de (N-méthylbenzylamino)-5 dithiole-1,2 thione-3 fondant à 138-140°C.

Le (N-méthylbenzylamino)-3 oxo-3 propionate d'éthyle peut être préparé par action de 36,3 g de N-méthylbenzylamine sur 45,2 g de chloroformylacétate d'éthyle en présence de 30,3 g de triéthylamine dans 300 cm3 de chlorure de méthylène à 20°C pendant 4 heures. Après hydrolyse du mélange réactionnel par 300 cm3 d'eau distillée, on lave la phase organique avec 300 cm3 d'eau distillée puis 300 cm3 d'une solution aqueuse d'acide chlorhydrique 1N et 2 fois 300 cm3 d'eau distillée. On sèche ensuite sur sulfate de magnésium, filtre et concentre à sec sous pression réduite (20 mm de mercure ; 2,7 kPa) à 40°C. On obtient ainsi 66 g de (N-méthylbenzylamino)-3 oxo-3 propionate d'éthyle sous forme d'une huile jaune pâle.

/Rf = 0,65 ; chromatographie sur couche mince de gel de silice ; solvant : acétate d'éthyle/.

EXEMPLE 6

A une solution de 3 g de (N-méthylphénylamino)-5 dithiole-1,2 thione-3 dans 43 cm3 d'acétone au reflux, on ajoute une solution de 2,66 g d'iodure de méthyle dans 8 cm3 d'acétone et on maintient le mélange réactionnel au reflux pendant 1 heure. Après refroidissement à une température voisine de 20°C, le produit insoluble formé est séparé par filtration et lavé avec 3 fois 10 cm3 d'acétone puis 3 fois 10 cm3 d'oxyde d'isopropyle.

Par recristallisation du produit ainsi obtenu dans un mélange de 50 cm3 d'éthanol et de 50 cm3 d'eau distillée, on obtient 4,3 g d'iodure de N-méthyl N-(méthylthio-5 dithiole-1,2 ylidène-3) phénylammonium fondant à 204°C avec décomposition.

La (N-méthylphénylamino)-5 dithiole-1,2 thione-3 peut être préparée par action de 127 g de pentasulfure de phosphore sur 85 g de (N-méthylphénylamino)-3 oxo-3 propionate d'éthyle dans 950 cm3 de pyridine au reflux pendant 1 heure. Après refroidissement à une température voisine de 20°C, le mélange réactionnel est hydrolysé par 7 litres d'eau distillée et extrait avec 3 fois 900 cm3 de chlorure de méthylène. Les phases organiques sont réunies et lavées avec 3 fois 500 cm3 d'ammoniaque 4N puis 500 cm3 d'eau distillée, séchées sur sulfate de magnésium,

filtrées et concentrées à sec sous pression réduite (20 mm de mercure ; 2,7 kPa) à 40°C. Le résidu obtenu est dissous dans 100 cm3 de chlorure de méthylène et la solution est versée sur 1000 g de gel de silice contenus dans une colonne de 6,5 cm de diamètre. On élue d'abord avec 1000 cm3 de chlorure de méthylène ; l'éluat correspondant est éliminé. On élue ensuite avec 16,5 litres de chlorure de méthylène ; l'éluat correspondant est concentré à sec sous pression réduite (20 mm de mercure ; 2,7 kPa) à 40°C. Le résidu est repris par 20 cm3 d'acétate d'éthyle et le produit insoluble est séparé par filtration et lavé avec 4 fois 5 cm3 d'acétate d'éthyle. On obtient ainsi 10,7 g de (N-méthylphénylamino)-5 dithiole-1,2 thione-3 fondant à 128°C.

Le (N-méthylphénylamino)-3 oxo-3 propionate d'éthyle peut être préparé par action de 42,6 g de N-méthylphénylamine sur 60 g de chloroformylacétate d'éthyle en présence de 40,3 g de triéthylamine dans 400 cm3 de chlorure de méthylène à une température voisine de 20°C pendant 2 heures. Après hydrolyse par 400 cm3 d'eau distillée, la phase organique est lavée successivement avec 250 cm3 d'une solution aqueuse d'acide chlorhydrique 1N, 4 fois 150 cm3 d'eau distillée, 310 cm3 d'une solution aqueuse à 0,45 % de bicarbonate de sodium et 300 cm3 d'eau distillée, séchée sur sulfate de magnésium, filtrée et concentrée à sec sous pression réduite (20 mm de mercure ; 2,7 kPa) à 40°C. On obtient ainsi 85 g de (N-méthylphénylamino)-3 oxo-3 propionate d'éthyle sous forme d'une huile orangée.

[Rf = 0,63 ; chromatographie sur couche mince de gel de silice ; solvant : acétate d'éthyle].

Le produit peut être distillé à 138-142°C sous 0,5 mm de mercure (0,067 kPa).

<u>EXEMPLE 7</u>

A une solution de 4 g de (N-méthylcyclohexylamino)-5 dithiole-1,2 thione-3 dans 55 cm3 d'acétone au reflux, on ajoute une solution de 3,45 g d'iodure de méthyle dans 11 cm3 d'acétone et on maintient le reflux pendant 1 heure. Après refroidissement à une température voisine de 20°C, le produit insoluble formé est séparé par filtration puis lavé

avec 3 fois 20 cm3 d'acétone et 2 fois 20 cm3 d'éther éthylique.

Par recristallisation du produit ainsi obtenu dans un mélange de 50 cm3 d'éthanol et de 50 cm3 d'eau distillée, on obtient 5 g d'iodure de N-méthyl N-(méthylthio-5 dithiole-1,2 ylidène-3) cyclohexylammonium fondant à 206°C.

La (N-méthylcyclohexylamino)-5 dithiole-1,2 thione-3 peut être préparée par action de 149 g de pentasulfure de phosphore sur 97 g de (N-méthylcyclohexylamino)-3 oxo-3 propionate d'éthyle dans 1110 cm3 de pyridine au reflux pendant 1 heure. Après refroidissement à une température voisine de 20°C, le mélange réactionnel est hydrolysé par 6,5 litres d'eau distillée puis extrait par 1300 puis 2 fois 950 cm3 de chlorure de méthylène. Les phases organiques sont réunies, lavées avec 3 fois 600 cm3 d'eau distillée, séchées sur sulfate de magnésium en présence de noir décolorant, filtrées et concentrées à sec sous pression réduite (20 mm de mercure ; 2,7 kPa) à 40°C. Le résidu est dissous dans 100 cm3 de chlorure de méthylène et la solution est versée sur 1500 g de gel de silice contenus dans une colonne de 9 cm de diamètre. On élue d'abord avec 2,6 litres de chlorure de méthylène ; l'éluat correspondant est éliminé. On élue ensuite avec 8,5 litres de chlorure de méthylène ; l'éluat correspondant est concentré à sec sous pression réduite (20 mm de mercure ; 2,7 kPa) à 40°C. Le résidu est repris par 100 cm3 d'acétate d'éthyle et le produit insoluble est séparé par filtration et lavé avec 4 fois 20 cm3 d'acétate d'éthyle puis 2 fois 20 cm3 d'éther éthylique. Après séchage, on obtient 20 g de (N-méthylcyclohexylamino)-5 dithiole-1,2 thione-3 fondant à 107°C.

Le (N-méthylcyclohexylamino)-3 oxo-3 propionate d'éthyle peut être préparé par action de 52,6 g de N-méthylcyclohexylamine sur 70 g de chloroformylacétate d'éthyle en présence de 47 g de triéthylamine dans 470 cm3 de chlorure de méthylène à une température voisine de 20°C pendant 2 heures. Après hydrolyse par 470 cm3 d'eau distillée, la phase organique est lavée avec 250 cm3 d'une solution aqueuse d'acide chlorhydrique 1N puis 4 fois 175 cm3 d'eau distillée, séchée sur

sulfate de magnésium en présence de noir décolorant, filtrée et concentrée à sec sous pression réduite (20 mm de mercure ; 2,7 kPa) à 40°C. On obtient ainsi 97 g de (N-méthylcyclohexylamino)-3 oxo-3 propionate d'éthyle sous forme d'une huile orange.

[Rf = 0,59 ; chromatographie sur couche mince de gel de silice ; solvant : acétate d'éthyle].

EXEMPLE 8

A une suspension de 10,15 g de (pyrrolidinyl-1)-5 dithiole-1,2 thione-3 dans 200 cm3 d'acétone on ajoute 10,65 g d'iodure de méthyle et chauffe le mélange réactionnel au reflux pendant 2 heures. Après refroidissement à une température voisine de 20°C, le produit insoluble est séparé par filtration et lavé avec 2 fois 30 cm3 d'acétone. Après recristallisation du produit ainsi obtenu dans 200 cm3 d'eau on obtient 14,8 g d'iodure de N-(méthylthio-5 dithiole-1,2 ylidène-3) pyrrolidinium fondant à 197°C.

La (pyrrolidinyl-1)-5 dithiole-1,2 thione-3 peut être préparée par action de 287,5 g de pentasulfure de phosphore sur 160 g de (pyrrolidinyl-1)-3 oxo-3 propionate d'éthyle dans 1600 cm3 de pyridine au reflux pendant 1 heure. Après refroidissement à une température voisine de 20°C, le mélange réactionnel est hydrolysé par 11 litres d'eau distillée et extrait successivement par 1600 puis 3 fois 500 cm3 de chlorure de méthylène. Les phases choronéthyléniques sont réunies, lavées avec 3 fois 500 cm3 d'eau distillée, séchées sur sulfate de magnésium en présence de noir décolorant, filtrées et concentrées à sec sous pression réduite (20 mm de mercure ; 2,7 kPa) à 40°C. Le résidu est repris par 100 cm3 de chlorure de méthylène et le produit insoluble est séparé par filtration et lavé avec 30 cm3 de chlorure de méthylène. Après séchage, on obtient ainsi 29 g de (pyrrolidinyl-1)-5 dithiole-1,2 thione-3 fondant à 230°C.

Le (pyrrolidinyl-1)-3 oxo-3 propionate d'éthyle peut être préparé par action de 94,4 g de pyrrolidine sur 200 g de chloroformylacétate d'éthyle en présence de 134,4 g de triéthylamine dans 1340 cm3

de chlorure de méthylène à une température voisine de 20°C pendant 2 heures. Après hydrolyse par 1340 cm3 d'eau distillée, la phase organique est lavée successivement avec 1100 cm3 d'une solution aqueuse d'acide chlorhydrique 1N, 3 fois 1100 cm3 d'eau distillée, séchée sur sulfate de magnésium, filtrée et concentrée à sec sous pression réduite (20 mm de mercure ; 2,7 kPa) à 40°C. On obtient ainsi 206,2 g de (pyrrolidinyl-1)-3 oxo-3 propionate d'éthyle sous forme d'une huile orange.

[Rf = 0,37 ; chromatographie sur couche mince de gel de silice ; solvant : acétate d'éthyle].

EXEMPLE 9

A une suspension de 3 g de (pyrrolidinyl-1)-5 dithiole-1,2 thione-3 dans 52 cm3 d'acétone au reflux, on ajoute goutte à goutte en 20 minutes une solution de 3,46 g d'iodoéthane dans 8 cm3 d'acétone puis on maintient le mélange réactionnel au reflux pendant 24 heures. Après refroidissement à une température voisine de 20°C, le produit insoluble formé est séparé par filtration et lavé avec trois fois 10 cm3 d'acétone puis avec deux fois 10 cm3 d'oxyde d'isopropyle.

Par recristallisation du produit ainsi obtenu dans 92 cm3 d'éthanol, on obtient 4,5 g d'iodure de N-(éthylthio-5 dithiole-1,2 ylidène-3) pyrrolidinium fondant à 165°C.

EXEMPLE 10

A une suspension de 2,8 g de (pyrrolidinyl-1)-5 dithiole-1,2 thione-3 et de 3,3 g d'iodure de sodium dans 37 cm3 d'acétone au reflux on ajoute goutte à goutte en 10 minutes une solution de 2,52 g de bromo-1 propane dans 5 cm3 d'acétone et on maintient le mélange réactionnel au reflux pendant 24 heures. Le produit insoluble formé est filtré à chaud, lavé successivement avec 5 cm3 d'acétone, avec deux fois 5 cm3 d'eau distillée puis avec deux fois 5 cm3 d'acétone.

Par recristallisation du produit ainsi obtenu dans 34 cm3 d'éthanol, on obtient 3,96 g d'iodure de N-(propylthio-5 dithiole-1,2 ylidène-3) pyrrolidinium fondant à 148°C.

EXEMPLE 11

A une suspension de 2,5 g de (pyrrolidinyl-1)-5 dithiole-1,2 thione-3 dans 43 cm3 d'acétone au reflux, on ajoute goutte à goutte en 20 minutes une solution de 4,18 g d'iodo-1 heptane dans 7 cm3 d'acétone et on maintient le mélange réactionnel au reflux pendant 30 heures. Après refroidissement à une température voisine de 20°C, le produit insoluble formé est séparé par filtration et lavé avec 3 fois 5 cm3 d'acétone, 4 fois 5 cm3 de sulfure de carbone puis par 2 fois 5 cm3 d'éther éthylique.

Par recristallisation du produit ainsi obtenu dans 50 cm3 de propanol-1, on obtient 4,3 g d'iodure de N-(heptylthio-5 dithiole-1,2 ylidène-3) pyrrolidinium fondant à 122°C.

EXEMPLE 12

A une suspension de 3,04 g de (pyrrolidinyl-1)-5 dithiole-1,2 thione-3 dans 50 cm3 d'acétone au reflux, on ajoute goutte à goutte en 5 minutes une solution de 3,76 g d'iodo-2 éthanol dans 10 cm3 d'acétone et on maintient le mélange réactionnel au reflux pendant 37 heures. Après refroidissement à une température voisine de 20°C, le produit insoluble formé est séparé par filtration et lavé avec 20 cm3 d'acétone.

Par recristallisation du produit ainsi obtenu dans 300 cm3 d'éthanol, on obtient 5,1 g d'iodure de N-[(hydroxy-2 éthyl)thio-5 dithiole-1,2 ylidène-3] pyrrolidinium fondant à 160-162°C.

EXEMPLE 13

A une suspension de 6,1 g de (pyrrolidinyl-1)-5 dithiole-1,2 thione-3 dans 95 cm3 d'acétone au reflux, on ajoute 7,41 g d'iodure de sodium puis on ajoute goutte à goutte une solution de 8,41 g de bromure de phénacyle dans 25 cm3 d'acétone. On maintient le mélange réactionnel au reflux pendant 1 heure puis on le refroidit à une température voisine de 20°C. Le produit insoluble formé est séparé par filtration et lavé successivement avec 30 cm3 d'acétone, 2 fois 40 cm3 d'eau distillée et 2 fois 30 cm3 d'acétone. Le produit brut obtenu est recristallisé dans un

mélange de 215 cm3 d'eau distillée et 215 cm3 d'éthanol puis agité pendant 15 minutes en présence de 200 cm3 d'eau distillée à une température voisine de 60°C. Après filtration et séchage, on obtient 10,9 g d'iodure de N-(phénacylthio -5 dithiole-1,2 ylidène-3) pyrrolidinium fondant à 179-180°C.

EXEMPLE 14

A une suspension de 3,04 g de (pyrrolidinyl-1)-5 dithiole-1,2 thione-3 et de 3,75 g d'iodure de sodium dans 45 cm3 d'acétone au reflux, on ajoute une solution de 2,12 g de chloracétone dans 5 cm3 d'acétone et on maintient au reflux pendant 1 heure. Après refroidissement à une température voisine de 20°C, le produit insoluble formé est séparé par filtration et lavé avec 15 cm3 d'acétone, 4 fois 25 cm3 d'eau distillée puis avec 30 cm3 d'acétone.

Par recristallisation du produit ainsi obtenu dans un mélange de 1600 cm3 d'éthanol et de 50 cm3 d'eau distillée, on obtient 3,6 g d'iodure de N-[(oxo-2 propyl)thio-5 dithiole-1,2 ylidène-3] pyrrolidinium fondant à 200-205°C.

EXEMPLE 15

A une suspension de 4,06 g de (pyrrolidinyl-1)-5 dithiole-1,2 thione-3 et de 4,8 g d'iodure de sodium dans 50 cm3 d'acétone au reflux, on ajoute une solution de 5 g de bromoacétate d'éthyle dans 10 cm3 d'acétone puis on maintient le milieu réactionnel au reflux pendant 18 heures. Après refroidissement à une température voisine de 10°C, le produit insoluble formé est séparé par filtration et lavé successivement avec 15 cm3 d'acétone, 4 fois 20 cm3 d'eau distillée puis 2 fois 15 cm3 d'acétone.

Par recristallisation du produit ainsi obtenu dans 200 cm3 d'éthanol, on obtient 6,8 g d'iodure de N-(éthoxycarbonylméthylthio-5 dithiole-1,2 ylidène-3) pyrrolidinium fondant à 168-170°C.

EXEMPLE 16

A une suspension de 2,5 g de (pyrrolidinyl-1)-5 dithiolè-1,2 thione-3 et de 1,7 g de chloroacétamide dans 44 cm3 d'acétone au reflux, on ajoute 3 g d'iodure de sodium et on maintient le reflux pendant 1 heure 30 minutes. Après refroidissement à une température voisine de 20°C, le produit insoluble formé est séparé par filtration et lavé successivement avec 3 fois 8 cm3 d'acétone, 3 fois 10 cm3 d'eau distillée, 2 fois 10 cm3 de sulfure de carbone et 2 fois 10 cm3 d'oxyde d'isopropyle.

Par recristallisation du produit ainsi obtenu dans 44 cm3 d'eau distillée, on obtient 4 g d'iodure de N-(carbamoylméthylthio-5 dithiole-1,2 ylidène-3) pyrrolidinium fondant à 222°C.

EXEMPLE 17

A une suspension de 2,5 g de (pyrrolidinyl-1)-5 dithiole-1,2 thione-3 et de 3 g d'iodure de sodium dans 34 cm3 d'acétone au reflux, on ajoute une solution de 1,38 g de chloroacétonitrile dans 10 cm3 d'acétone et on maintient le mélange réactionnel au reflux pendant 20 heures. Après refroidissement à une température voisine de 20°C, le produit insoluble formé est séparé par filtration et lavé successivement avec 3 fois 5 cm3 d'acétone, 3 fois 5 cm3 d'eau distillée puis 2 fois 5 cm3 d'acétone.

Par recristallisation du produit ainsi obtenu dans 60 cm3 d'eau distillée, on obtient 3,25 g d'iodure de N-(cyanométhylthio-5 dithiole-1,2 ylidène-3) pyrrolidinium fondant à 191°C.

EXEMPLE 18

A une suspension de 7,4 g de chlorhydrate de diéthylamino-2 chloro-1 éthane, de 4,45 g de (pyrrolidinyl-1)-5 dithiole-1,2 thione-3 et de 9,9 g d'iodure de sodium dans un mélange de 110 cm3 d'acétone et de 25 cm3 de diméthylformamide au reflux, on ajoute goutte à goutte en 5 minutes 4,35 g de triéthylamine et on maintient le mélange réactionnel

au reflux pendant 6 heures puis filtre à une température voisine de 50°C pour séparer le produit insoluble formé.

Par recristallisation du produit ainsi obtenu dans 250 cm3 d'éthanol, on obtient 2,4 g de chlorhydrate d'iodure de N-[(diéthylamino-2 éthyl)thio-5 dithiole-1,2 ylidène-3] pyrrolidinium fondant à 200-204°C.

EXEMPLE 19

A une suspension de 3 g de (pyrrolidinyl-1)-5 dithiole-1,2 thione-3 et de 3,6 g d'iodure de sodium dans 43 cm3 d'acétone au reflux, on ajoute goutte à goutte en 10 minutes une solution de 2,66 g de bromure d'allyle dans 10 cm3 d'acétone et on maintient le mélange réactionnel au reflux pendant 1 heure. Après refroidissement à une température voisine de 20°C, le produit insoluble formé est séparé par filtration puis lavé avec 3 fois 10 cm3 d'acétone, 4 fois 10 cm3 d'eau distillée et 3 fois 10 cm3 d'éther éthylique.

Par recristallisation du produit ainsi obtenu dans 96 cm3 d'éthanol, on obtient 3,36 g d'iodure de N-(allylthio-5 dithiole-1,2 ylidène-3) pyrrolidinium fondant à 160°C.

EXEMPLE 20

A une suspension de 3,7 g de chlorhydrate de chlorométhyl-2 pyridine, de 3,04 g de (pyrrolidinyl-1)-5 dithiole-1,2 thione-3 et de 6,75 g d'iodure de sodium dans un mélange de 50 cm3 d'acétone et de 15 cm3 de diméthylformamide au reflux, on ajoute goutte à goutte en 5 minutes une solution de 2,28 g de triéthylamine dans 25 cm3 d'acétone et on maintient le mélange réactionnel au reflux pendant 3 heures. Après refroidissement à une température voisine de 20°C, le produit insoluble formé est séparé par filtration, lavé successivement avec 2 fois 15 cm3 d'acétone, 2 fois 10 cm3 d'eau distillée et 1 fois 15 cm3 d'acétone.

Par recristallisation du produit ainsi obtenu dans 110 cm3 d'éthanol, on obtient 2 g d'iodure de N-[(pyridyl-2) méthylthio-5 dithiole-1,2 ylidène-3] pyrrolidinium fondant à 162-163°C.

EXEMPLE 21

A une suspension de 2,5 g de (pyrrolidinyl-1)-5 dithiole-1,2 thione-3 et de 3 g d'iodure de sodium dans 37 cm3 d'acétone au reflux, on ajoute goutte à goutte en 15 minutes une solution de 2,5 g de chlorométhyl-3 pyridine dans 7 cm3 d'acétone et on maintient le mélange réactionnel au reflux pendant 1 heure 30 minutes. Après refroidissement à une température voisine de 20°C, le produit insoluble formé est séparé par filtration, lavé successivement avec 3 fois 5 cm3 d'acétone, 2 fois 5 cm3 d'eau distillée et 2 fois 5 cm3 d'oxyde d'isopropyle.

Par recristallisation du produit ainsi obtenu dans 73 cm3 d'eau distillée, on obtient 3,5 g d'iodure de N-[(pyridinyl-3) méthylthio-5 dithiole-1,2 ylidène-3] pyrrolidinium fondant à 178°C.

EXEMPLE 22

Une suspension de 2 g de (pyrrolidinyl-1)-5 dithiole-1,2 thione-3, de 2,4 g de chlorhydrate de chlorométhyl-4 pyridine et de 2,2 g d'iodure de sodium dans 36 cm3 d'acétone est chauffée au reflux pendant 2 heures. Après refroidissement à une température voisine de 20°C, le produit insoluble formé est séparé par filtration et lavé avec 3 fois 10 cm3 d'acétone puis avec 3 fois 10 cm3 de sulfure de carbone.

Par recristallisation du produit ainsi obtenu dans 480 cm3 d'éthanol, on obtient 2,8 g d'hémiiodhydrate d'iodure de N-[(pyridyl-4) méthylthio-5 dithiole-1,2 ylidène-3] pyrrolidinium fondant vers 170°C.

EXEMPLE 23

A une suspension de 4,45 g de diméthylamino-5 dithiole-1,2 thione-3, de 6,15 g de chlorhydrate de chlorométhyl-3 pyridine et de 11,25 g d'iodure de sodium dans un mélange de 125 cm3 d'acétone et 25 cm3 de diméthylformamide, on ajoute 5,25 cm3 de triéthylamine. Le mélange réactionnel est chauffé au reflux pendant 6 heures puis filtré bouillant. Le produit insoluble formé est recueilli et lavé successivement avec 40 cm3 d'acétone, 40 cm3 d'eau et 2 fois 25 cm3 d'acétone.

Par recristallisation du produit ainsi obtenu dans 45 cm3 d'eau, on obtient 3,4 g d'iodure de N-[(pyridyl-3) méthylthio-5 dithiole-1,2 ylidène-3] diméthylammonium fondant à 185°C.

EXEMPLE 24

A une suspension de 3,26 g de (pipéridinyl-1)-5 dithiole-1,2 thione-3 dans 50 cm3 d'acétone au reflux, on ajoute une solution de 6,4 g d'iodure de méthyle dans 10 cm3 d'acétone et on maintient le mélange réactionnel au reflux pendant 2 heures. Après refroidissement à une température voisine de 20°C, le produit insoluble formé est séparé par filtration et lavé avec 10 cm3 d'acétone.

Par recristallisation du produit ainsi obtenu dans 50 cm3 d'éthanol, on obtient 3,9 g d'iodure de N-(méthylthio-5 dithiole-1,2 ylidène-3) pipéridinium fondant à 149-150°C.

La (pipéridinyl-1)-5 dithiole-1,2 thione-3 peut être préparée par action de 127 g de pentasulfure de phosphore sur 71 g de (pipéridinyl-1)-3 oxo-3 propionate d'éthyle dans 720 cm3 de pyridine au reflux pendant 1 heure. Le mélange réactionnel est ensuite refroidi à une température voisine de 20°C, dilué par 6 litres d'eau et extrait par 6 fois 500 cm3 de chlorure de méthylène. Les phases organiques sont réunies, lavées avec 3 fois 500 cm3 d'eau distillée, séchées sur sulfate de magnésium, filtrées et concentrées à sec sous pression réduite (20 mm de mercure ; 2,7 kPa) à 40°C. Le résidu obtenu est repris par 90 cm3 de diméthylformamide et le produit insoluble est séparé par filtration, lavé avec 5 cm3 de diméthylformamide puis 2 fois 15 cm3 d'éther éthylique.

Par recristallisation du produit ainsi obtenu dans 600 cm3 d'acétonitrile, on obtient 9,5 g de (pipéridinyl-1)-5 dithiole-1,2 thione-3 fondant à 194°C.

Le (pipéridinyl-1)-3 oxo-3 propionate d'éthyle peut être préparé par action de 34 g de pipéridine sur 60 g de chloroformylacétate d'éthyle en présence de 40,4 g de triéthylamine dans 400 cm3 de chlorure de méthylène à une température voisine de 20°C pendant 3 heures.

Après hydrolyse par 400 cm3 d'eau, la phase organique est lavée successivement avec 400 cm3 d'une solution aqueuse d'acide chlorhydrique 1N, 2 fois 400 cm3 d'eau distillée, 400 cm3 d'une solution aqueuse à 2 % de bicarbonate de sodium puis avec 400 cm3 d'eau distillée, séchée sur sulfate de magnésium, filtrée et concentrée à sec sous pression réduite (20 mm de mercure ; 2,7 kPa) à 40°C. On obtient ainsi 71 g de (pipéridinyl-1)-3 oxo-3 propionate d'éthyle sous forme d'une huile orange.

[Rf = 0,45 ; chromatographie sur couche mince de gel de silice ; solvant : acétate d'éthyle].

EXEMPLE 25

A une solution de 2,5 g de (perhydroazépinyl-1)-5 dithiole-1,2 thione-3 dans 38 cm3 d'acétone au reflux, on ajoute une solution de 2,31 g d'iodure de méthyle dans 6 cm3 d'acétone et on maintient le mélange réactionnel au reflux pendant 1 heure. Après refroidissement à une température voisine de 20°C, le produit insoluble formé est séparé par filtration et lavé successivement avec 3 fois 5 cm3 d'acétone, 2 fois 5 cm3 de sulfure de carbone et 2 fois 5 cm3 d'éther éthylique.

Par recristallisation du produit ainsi obtenu dans 16 cm3 d'éthanol, on obtient 3,6 g d'iodure de N-(méthylthio-5 dithiole-1,2 ylidène-3) perhydroazépinium fondant à 130°C.

La (perhydroazépinyl-1)-5 dithiole-1,2 thione-3 peut être préparée par action de 91,4 g de pentasulfure de phosphore sur 59 g de (perhydroazépinyl-1)-3 oxo-3 propionate d'éthyle dans 680 cm3 de pyridine au reflux pendant 1 heure. Le mélange réactionnel est ensuite refroidi à une température voisine de 20°C, dilué par 6860 cm3 d'eau distillée et extrait par 780 cm3 puis 2 fois 560 cm3 de chlorure de méthylène. Les phases organiques sont réunies, lavées successivement avec 3 fois 370 cm3 d'ammoniaque 4N puis 370 cm3 d'eau distillée, séchées sur sulfate de magnésium, filtrées et concentrées à sec sous pression réduite (20 mm de mercure ; 2,7 kPa) à 40°C. Le résidu est repris par 25 cm3 d'acétonitrile ; le produit insoluble est séparé par filtration, lavé avec 3 fois 10 cm3 d'acétonitrile. Le produit ainsi obtenu est dissous

dans 50 cm3 de chlorure de méthylène et la solution obtenue est versée sur 200 g de gel de silice contenus dans une colonne de 4 cm de diamètre. On élue d'abord avec 900 cm3 de chlorure de méthylène ; l'éluat correspondant est éliminé. On élue ensuite avec 6100 cm3 de chlorure de méthylène ; l'éluat correspondant est concentré à sec sous pression réduite (20 mm de mercure ; 2,7 kPa) à 40°C. On obtient ainsi 8,1 g de (perhydroazépinyl-1)-5 dithiole-1,2 thione-3 fondant à 120°C.

Le (perhydroazépinyl-1)-3 oxo-3 propionate d'éthyle peut être préparé par action de 33 g de perhydroazépine sur 50 g de chloroformylacétate d'éthyle en présence de 33,6 g de triéthylamine dans 333 cm3 de chlorure de méthylène à une température voisine de 20°C pendant 2 heures. Après hydrolyse par 333 cm3 d'eau distillée, la phase organique est lavée successivement avec 270 cm3 d'une solution aqueuse d'acide chlorhydrique 1N, 250 cm3 puis 2 fois 120 cm3 d'eau distillée, 260 cm3 d'une solution aqueuse de bicarbonate de sodium à 0,4 % et 250 cm3 d'eau distillée, séchée sur sulfate de magnésium en présence de noir décolorant, filtrée et concentrée à sec sous pression réduite (20 mm de mercure ; 2,7 kPa) à 40°C. On obtient ainsi 60 g de (perhydroazépinyl-1)-3 oxo-3 propionate d'éthyle sous forme d'une huile orange.

[Rf = 0,63 ; chromatographie sur couche mince de gel de silice ; solvant : acétate d'éthyle].

EXEMPLE 26

A une suspension de 4,8 g de morpholino-5 dithiole-1,2 thione-3 dans 80 cm3 d'acétone au reflux, on ajoute une solution de 9,35 g d'iodure de méthyle dans 20 cm3 d'acétone et on maintient le mélange réactionnel au reflux pendant 3 heures. Après refroidissement à une température voisine de 10°C, le produit insoluble formé est séparé par filtration et lavé avec 20 cm3 d'acétone.

Par recristallisation du produit ainsi obtenu dans un mélange de 50 cm3 d'éthanol et 50 cm3 d'eau distillée, on obtient 7,2 g d'iodure

de N-(méthylthio-5 dithiole-1,2 ylidène-3) morpholinium fondant à 228-230°C.

La morpholino-5 dithiole-1,2 thione-3 peut être préparée par action de 53,6 g de pentasulfure de phosphore sur 30 g de morpholino-3 oxo-3 propionate d'éthyle dans 300 cm3 de pyridine au reflux pendant 1 heure. Le mélange réactionnel est ensuite refroidi à une température voisine de 20°C, hydrolysé par 3 litres d'eau et extrait avec 500 cm3 de chlorure de méthylène. Le produit insoluble formé est séparé par filtration, lavé avec 2 fois 20 cm3 d'oxyde d'isopropyle et séché. On obtient ainsi 3,1 g de morpholino-5 dithiole-1,2 thione-3 fondant à 248-250°C. Le filtrat est ensuite lavé avec 4 fois 300 cm3 d'eau distillée, séché sur sulfate de magnésium, filtré et concentré à sec sous pression réduite (20 mm de mercure ; 2,7 kPa) à 40°C. Le résidu obtenu est repris par 30 cm3 de diméthylformamide et le produit insoluble formé est séparé par filtration, lavé avec 5 cm3 de diméthylformamide puis avec 2 fois 5 cm3 de chlorure de méthylène. Après séchage, on obtient un nouveau lot de 2 g de morpholino-5 dithiole-1,2 thione-3 fondant à 248-250°C.

Le morpholino-3 oxo-3 propionate d'éthyle peut être préparé par action de 17,4 g de morpholine sur 30,1 g de chloroformylacétate d'éthyle en présence de 20,2 g de triéthylamine dans 200 cm3 de chlorure de méthylène à une température voisine de 20°C pendant 2 heures 30 minutes. Après hydrolyse par 200 cm3 d'eau distillée, la phase organique est lavée successivement avec 200 cm3 d'eau distillée, 200 cm3 d'une solution aqueuse d'acide chlorhydrique 1N, 2 fois 200 cm3 d'eau distillée, 200 cm3 d'une solution aqueuse à 2 % de bicarbonate de sodium, puis 200 cm3 d'eau distillée, séchée sur sulfate de magnésium, filtrée et concentrée à sec sous pression réduite (20 mm de mercure ; 2,7 kPa) à 40°C. On obtient ainsi 30 g de morpholino-3 oxo-3 propionate d'éthyle fondant à 60°C.

EXEMPLE 27

A une solution de 4,02 g de morpholino-5 dithiole-1,2 thione-3 dans 80 cm3 d'acétone, on ajoute 2,8 g de chloracétamide et 4,95 g d'iodure de sodium et on porte le mélange réactionnel au reflux pendant 3 heures. Après refroidissement à une température voisine de 20°C, on filtre le produit insoluble formé et le lave successivement avec 20 cm3 d'acétone, 3 fois 20 cm3 d'eau distillée et 40 cm3 d'acétone.

Après recristallisation dans un mélange de 480 cm3 d'éthanol et 120 cm3 d'eau distillée, on obtient 4,3 g d'iodure de N-(carbamoylméthylthio-5 dithiole-1,2 ylidène-3) morpholinium fondant à 244-247°C.

EXEMPLE 28

A une solution de 3,4 g de phénylamino-5 dithiole-1,2 thione-3 dans 30 cm3 d'acétone au reflux, on ajoute une solution de 4,25 g d'iodure de méthyle dans 5 cm3 d'acétone et on maintient le reflux pendant 1 heure. Après refroidissement à une température voisine de 20°C, le produit insoluble formé est séparé par filtration et lavé avec 5 cm3 d'acétone.

Par recristallisation du produit ainsi obtenu dans 300 cm3 d'éthanol, on obtient 4 g d'iodhydrate de méthylthio-5 phénylimino-3 dithiole-1,2 fondant à 164-165°C.

La phénylamino-5 dithiole-1,2 thione-3 peut être préparée par action de 90 g de phénylamino-3 oxo-3 propionate d'éthyle sur 146 g de pentasulfure de phosphore dans 900 cm3 de dioxanne au reflux pendant 20 minutes. Après refroidissement à une température voisine de 20°C, le mélange réactionnel est versé dans un mélange de 5,5 litres d'eau, 650 cm3 d'ammoniaque concentré (d = 0,92) et 1,5 litre de chlorure de méthylène. On agite ensuite pendant 20 minutes. La phase aqueuse est alors décantée et lavée 2 fois avec 700 cm3 de chlorure de méthylène. Les phases chlorométhyléniques sont réunies, séchées sur sulfate de sodium, filtrées et concentrées à sec sous pression réduite

(20 mm de mercure ; 2,7 kPa) à 40°C. Le résidu obtenu est repris par 65 cm3 de chlorure de méthylène ; le produit insoluble est séparé par filtration puis lavé 4 fois avec 15 cm3 de chlorure de méthylène et 2 fois avec 20 cm3 d'oxyde d'isopropyle. On obtient ainsi 19 g de phénylamino-5 dithiole-1,2 thione-3 fondant à 135°C.

Le phénylamino-3 oxo-3 propionate d'éthyle peut être préparé selon la méthode décrite par F.D. CHATTAWAY et coll., J. Chem. Soc. 97, 939 (1910).

EXEMPLE 29

Une solution de 9 g de phénylamino-5 dithiole-1,2 thione-3, de 4,15 g d'acide chloracétique et de 6,6 g d'iodure de sodium dans 135 cm3 d'acétone est chauffée au reflux pendant 35 minutes. Après refroidissement à une température voisine de 20°C le produit insoluble formé est séparé par filtration et lavé successivement avec 3 fois 15 cm3 d'acétone, 3 fois 10 cm3 d'eau puis 3 fois 10 cm3 d'acétone. Par recristallisation de ce produit brut dans 470 cm3 de méthanol on obtient 7,1 g d'iodhydrate de l'acide (phénylimino-5 dithiole-1,2 yl-3 thio) acétique fondant vers 205°C avec décomposition.

On peut préparer la base correspondante de la manière suivante : l'iodhydrate obtenu précédemment est mis en suspension dans 70 cm3 d'eau distillée. On ajoute à cette suspension 2,93 g de bicarbonate de sodium : on obtient alors une solution à pH 7 avec un très faible insoluble que l'on sépare par filtration. Le filtrat obtenu est ensuite acidifié à pH 5 par addition de 16,5 cm3 d'une solution aqueuse d'acide chlorhydrique 1N. Le produit qui précipite est séparé par filtration puis lavé 9 fois avec 10 cm3 d'eau. Après séchage on obtient 3,7 g d'acide (phénylimino-5 dithiole-1,2 yl-3 thio) acétique fondant à 147°C.

EXEMPLE 30

A une solution de 4 g de phénylamino-5 dithiole-1,2 thione-3 dans 70 cm3 d'acétone, on ajoute 4,5 g de bromoacétate d'éthyle et on chauffe le mélange réactionnel au reflux pendant 24 heures. Après

refroidissement à une température voisine de 20°C, le produit insoluble formé est séparé par filtration et lavé avec 3 fois 5 cm3 d'acétone. Les filtrats sont réunis et concentrés à sec sous pression réduite (20 mm de mercure ; 2,7 kPa) à 40°C et le résidu est repris par 15 cm3 d'éthanol. Le produit insoluble est séparé par filtration et lavé avec 3 fois 10 cm3 d'éthanol puis avec 2 fois 10 cm3 d'éther éthylique.

Par recristallisation du produit ainsi obtenu dans 36 cm3 de méthyléthylacétone, on obtient 2,8 g de bromhydrate de (phénylimino-3 dithiol-1,2 yl-5 thio) acétate d'éthyle fondant à 90°C.

EXEMPLE 31

A une solution de 3,4 g de phénylamino-5 dithiole-1,2 thione-3 dans 60 cm3 d'acétone, on ajoute 2,15 g de chloroacétamide et 3,75 g d'iodure de sodium. Le mélange réactionnel est chauffé au reflux pendant 1 heure. Après refroidissement à une température voisine de 20°C, on filtre le produit insoluble formé et le lave successivement avec 20 cm3 d'acétone, 3 fois 20 cm3 d'eau distillée et 20 cm3 d'acétone.

Par recristallisation du produit ainsi obtenu dans 500 cm3 d'éthanol, on obtient 3,5 g d'iodhydrate de (phénylimino-3 dithiole-1,2 yl-5 thio) acétamide fondant à 174-175°C.

EXEMPLE 32

Une solution de 13,5 g de phénylamino-5 dithiole-1,2 thione-3, 7,08 g de chlorure de diméthylcarbamoyle et 9,9 g d'iodure de sodium dans 205 cm3 d'acétone anhydre est chauffée au reflux pendant 24 heures. Après refroidissement à une température voisine de 20°C, le produit insoluble formé est séparé par filtration et lavé successivement avec 3 fois 25 cm3 d'acétone puis 3 fois 25 cm3 d'eau. On obtient ainsi 12,1 g d'iodhydrate de diméthylcarbamoylthio-5 phénylimino-3 dithiole-1,2 fondant à 214°C.

On peut préparer la base correspondante de la manière suivante : Le sel obtenu précédemment est mis en suspension dans 120 cm3 d'eau.

On ajoute alors, sous agitation, 2,9 g de bicarbonate de sodium puis 250 cm3 de chlorure de méthylène. Après 20 minutes d'agitation la phase aqueuse est décantée et lavée avec 50 cm3 de chlorure de méthylène. Les phases chlorométhyléniques sont réunies, séchées sur sulfate de sodium, filtrées et concentrées à sec sous pression réduite (20 mm de mercure ; 2,7 kPa) à 40°C.

Par recristallisation du résidu ainsi obtenu dans 55 cm3 d'acétonitrile, on obtient 8 g de diméthylcarbamoylthio-5 phénylimino-3 dithiole-1,2 fondant à 134°C.

EXEMPLE 33

A une suspension de 9 g de phénylamino-5 dithiole-1,2 thione-3 et de 23,9 g de chlorhydrate de chlorocarbonyl-1 méthyl-4 pipérazine dans 90 cm3 de chlorure de méthylène anhydre maintenue à une température voisine de 20°C, on ajoute goutte à goutte sous agitation et en 15 minutes, 16,2 g de triéthylamine puis 32 cm3 de pyridine anhydre. Le mélange réactionnel est ensuite chauffé au reflux pendant 3 heures. Après refroidissement à une température voisine de 20°C le mélange réactionnel est versé dans 400 cm3 d'eau, la phase aqueuse est décantée et lavée 2 fois avec 100 cm3 de chlorure de méthylène. Les phases chlorométhyléniques sont réunies, lavées 4 fois avec 100 cm3 d'eau distillée, séchées sur sulfate de sodium, filtrées et concentrées à sec sous pression réduite (20 mm de mercure ; 2,7 kPa) à 40°C. Le résidu obtenu est repris par 20 cm3 d'acétone ; le produit insoluble est séparé par filtration puis lavé 3 fois avec 6 cm3 d'acétone.

Par recristallisation du produit ainsi obtenu dans 235 cm3 d'acétonitrile on obtient 8,3 g de (méthyl-4 pipérazinyl-1) carbonylthio-5 phénylimino-3 dithiole-1,2 fondant à 146°C.

EXEMPLE 34

A une suspension de 3 g de phénylamino-5 dithiole-1,2 thione-3 dans 53 cm3 d'acétone, on ajoute 3,3 g d'iodure de sodium puis 3,6 g de N-chlorocarbonylmorpholine. On chauffe le mélange réactionnel au

reflux pendant 20 heures. Après refroidissement à une température voisine de 20°C, le produit insoluble formé est séparé par filtration puis lavé successivement avec 2 fois 10 cm3 d'acétone, 4 fois 10 cm3 d'eau distillée et 3 fois 10 cm3 d'éther éthylique. Par recristallisation du produit ainsi obtenu dans un mélange de 80 cm3 d'éthanol et de 80 cm3 d'eau distillée, on obtient 4,4 g d'un mélange d'iodhydrate et de chlorhydrate de morpholinocarbonylthio-5 phénylimino-3 dithiole-1,2 fondant vers 140°C. On agite pendant 30 minutes une suspension du mélange de sels ainsi obtenu dans un mélange de 60 cm3 de chlorure de méthylène et de 40 cm3 d'eau distillée additionnée de 1 g de bicarbonate de sodium. On sépare la phase organique par décantation et lave la phase aqueuse avec 60 cm3 de chlorure de méthylène. Les phases chlorométhyléniques sont réunies et lavées avec 40 cm3 d'eau distillée, séchées sur sulfate de magnésium en présence de noir décolorant, filtrées et concentrées à sec sous pression réduite (20 mm de mercure ; 2,7 kPa) à 40°C.

Par recristallisation du produit ainsi obtenu dans 250 cm3 d'éthanol, on obtient 2,76 g de morpholinocarbonylthio-5 phénylimino-3 dithiole-1,2 fondant à 162°C.

EXEMPLE 35

Une solution de 11,3 g de phénéthylamino-5 dithiole-1,2 thione-3 et de 12,8 g d'iodure de méthyle dans 225 cm3 d'acétone est chauffée au reflux pendant 1 heure. Après refroidissement à une température voisine de 20°C, le produit insoluble formé est séparé par filtration et lavé avec 2 fois 20 cm3 d'acétone puis séché. Par recristallisation du produit brut ainsi obtenu dans 730 cm3 d'eau, on obtient 12,3 g d'iodhydrate de phénéthylimino-3 méthylthio-5 dithiole-1,2 fondant à 172°C.

La phénéthylamino-5 dithiole-1,2 thione-3 peut être préparée par action de 80,9 g de phénéthylamino-3 oxo-3 propionate d'éthyle sur 114 g de pentasulfure de phosphore dans 810 cm3 de dioxanne au reflux pendant 1 heure. Après refroidissement à une température voisine de 20°C

le mélange réactionnel est versé dans un mélange de 4 litres d'eau, 0,7 litre d'ammoniaque concentré (d = 0,92) et 1,5 litre de chlorure de méthylène et agité pendant 20 minutes. La phase aqueuse est alors décantée et lavée avec 1 litre de chlorure de méthylène. Les phases chlorométhyléniques sont réunies, séchées sur sulfate de sodium, filtrées et concentrées à sec sous pression réduite (20 mm de mercure ; 2,7 kPa) à 40°C. Le résidu obtenu est dissous dans 1,4 litre d'un mélange chlorure de méthylène-cyclohexane (50-50 en volumes) et la solution obtenue est versée sur 1400 g de gel de silice contenus dans une colonne de 9 cm de diamètre. On élue avec 7 litres d'un mélange chlorure de méthylène-cyclohexane (50-50 en volumes) puis avec 10 litres de chlorure de méthylène pur. Ces éluats sont éliminés. On élue ensuite avec 15 litres de chlorure de méthylène pur puis avec 3 litres d'un mélange chlorure de méthylène-méthanol (99-1 en volumes). Ces éluats sont réunis et concentrés à sec sous pression réduite (20 mm de mercure ; 2,7 kPa) à 40°C. On obtient ainsi 27 g de phénéthylamino-5 dithiole-1,2 thione-3 sous forme d'une huile brune.

[Rf = 0,14 ; chromatographie sur couche mince de gel de silice ; solvant : chlorure de méthylène].

Le phénéthylamino-3 oxo-3 propionate d'éthyle peut être préparé par action de 42,3 g de phénéthylamine sur 52,7 g de chloroformylacétate d'éthyle dans 425 cm3 de chlorure de méthylène anhydre, en présence de 35,4 g de triéthylamine, pendant 2 heures 30 minutes à une température voisine de 20°C. On ajoute alors 250 cm3 d'eau au mélange réactionnel puis la phase chlorométhylénique est décantée et lavée successivement avec 250 cm3 d'une solution aqueuse d'acide chlorhydrique 1N, 250 cm3 d'eau, 250 cm3 d'une solution aqueuse de soude 1N et 3 fois 250 cm3 d'eau. Après séchage sur sulfate de sodium la phase chlorométhylénique est filtrée et concentrée à sec sous pression réduite (20 mm de mercure ; 2,7 kPa) à 40°C. On obtient ainsi 67,9 g de phénéthylamino-3 oxo-3 propionate d'éthyle fondant à 60°C.

EXEMPLE 36

A une solution de 1,9 g d'éthylamino-5 dithiole-1,2 thione-3 dans 30 cm3 d'acétone au reflux, on ajoute une solution de 4,25 g d'iodure de méthyle dans 5 cm3 d'acétone et on maintient le mélange réactionnel au reflux pendant 2 heures 20 minutes. Après refroidissement à une température voisine de 20°C, on filtre le produit insoluble formé et le lave avec 10 cm3 d'acétone.

Après recristallisation dans 50 cm3 d'éthanol, on obtient 0,8 g d'iodhydrate d'éthylimino-3 méthylthio-5 dithiole-1,2 fondant à 190°C.

L'éthylamino-5 dithiole-1,2 thione-3 peut être obtenue par action de 65 g d'éthylamino-3 oxo-3 propionate d'éthyle sur 135 g de pentasulfure de phosphore dans 970 cm3 de pyridine au reflux pendant 3 heures. Après refroidissement à une température voisine de 20°C, le mélange réactionnel est hydrolysé par 6 litres d'eau distillée puis extrait avec 1 litre puis 3 fois 500 cm3 de chlorure de méthylène. Les phases chlorométhyléniques sont réunies, lavées avec 3 fois 800 cm3 d'eau distillée, séchées sur sulfate de magnésium, filtrées et concentrées à sec sous pression réduite (20 mm de mercure ; 2,7 kPa) à 40°C. Le résidu est dissous dans 100 cm3 de chlorure de méthylène et versé sur 2 kg de gel de silice contenus dans une colonne de 10 cm de diamètre. On élue d'abord avec 18 litres de chlorure de méthylène ; l'éluat correspondant est éliminé. On élue ensuite avec 3 litres d'un mélange de chlorure de méthylène et d'acétate d'éthyle (80-20 en volumes) ; l'éluat correspondant est concentré à sec sous pression réduite (20 mm de mercure ; 2,7 kPa) à 40°C.

On obtient ainsi 2 g d'éthylamino-5 dithiole-1,2 thione-3 sous forme d'une huile brune.

[Rf = 0,70 ; chromatographie sur couche mince de gel de silice ; solvant : acétate d'éthyle].

L'éthylamino-3 oxo-3 propionate d'éthyle peut être obtenu par action de 27 g d'éthylamine sur 90,5 g de chloroformylacétate d'éthyle en présence de 60,5 g de triéthylamine dans 600 cm3 de chlorure de

méthylène à une température voisine de 20°C pendant 4 heures. Après hydrolyse par 400 cm3 d'eau distillée, le mélange réactionnel est décanté et la phase chlorométhylénique est lavée successivement avec 2 fois 150 cm3 d'eau distillée, 100 cm3 d'une solution aqueuse d'acide chlorhydrique 1N puis 2 fois 150 cm3 d'eau distillée. La phase organique est ensuite séchée sur sulfate de magnésium, filtrée et concentrée à sec sous pression réduite (20 mm de mercure ; 2,7 kPa) à 40°C.

On obtient ainsi 65 g d'éthylamino-3 oxo-3 propionate d'éthyle fondant à 40°C.

EXEMPLE 37 -

A une suspension de 4,06 g de (pyrrolidinyl-1)-5 dithiole-1,2 thione-3 et de 4,9 g d'iodure de sodium dans 70 cm3 d'acétone, on ajoute 3,7 g de N,N-diméthylchloracétamide et on maintient le mélange réactionnel au reflux pendant 1 heure. Après refroidissement, le produit insoluble est séparé par filtration et lavé successivement avec 3 fois 20 cm3 d'acétone, 3 fois 20 cm3 d'eau puis 3 fois 20 cm3 d'éther éthylique. Le produit obtenu est ensuite recristallisé une première fois dans 150 cm3 de méthanol puis une seconde fois dans 340 cm3 d'éthanol. On obtient ainsi 4,8 g d'iodure de N-(diméthylcarbamoylméthylthio-5 dithiole-1,2 ylidène-3] pyrrolidinium fondant à 152°C.

EXEMPLE 38 -

A une suspension de 3 g de (pyrrolidinyl-1)-5 dithiole-1,2 thione-3 et de 3,3 g d'iodure de sodium dans 54 cm3 d'acétone, on ajoute 6,38 g de bromhydrate de bromoacétyl-3 pyridine et on maintient le mélange réactionnel au reflux pendant 12 heures. Après refroidissement à une température voisine de 20°C, le produit insoluble est filtré et lavé successivement avec 3 fois 20 cm3 d'acétone puis 10 cm3 d'eau distillée. Par recristallisation du produit ainsi obtenu dans 100 cm3 de méthanol, on obtient 3,55 g de bromhydrate de bromure de [(pyridyl-3) carbonylméthylthio-5 dithiole-1,2 ylidène-3] pyrrolidinium fondant à 155°C avec décomposition.

On peut préparer la base correspondante de la manière suivante : le sel obtenu précédemment est mis en suspension dans 100 cm3 d'eau distillée. On ajoute alors, sous agitation, 2,6 g de bicarbonate de sodium et 155 cm3 de chlorure de méthylène. Après 5 minutes d'agitation, la phase organique est décantée, lavée avec 100 cm3 d'eau distillée, séchée sur sulfate de magnésium en présence de noir décolorant, filtrée et concentrée à sec sous pression réduite (20 mm de mercure ; 2,7 kPa) à 40°C.

Par recristallisation du résidu ainsi obtenu dans 700 cm3 d'éthanol, on obtient 5,9 g d'(acétyl-4 pipérazinyl) carbonylthio-5 phénylimino-3 dithiole-1,2 fondant à 193°C.

EXEMPLE 41 -

A une suspension de 9,85 g de morpholino-5 dithiole-1,2 thione-3 dans 200 cm3 d'éthanol, on ajoute 10,5 g de chlorure de phénacyle et on maintient le mélange réactionnel au reflux pendant 3 heures. Après refroidissement à une température voisine de 10°C, le produit insoluble formé est séparé par filtration et lavé avec 30 cm3 d'éthanol. Après séchage on obtient 14,3 g de chlorure de N-(phénacylthio-5 dithiole-1,2 ylidène-3) morpholinium fondant à 138°C, solvaté par 9 % d'éthanol.

Le produit ainsi préparé sous forme de chlorure peut être transsalifié en sulfate acide de la manière suivante :

A une solution de 0,75 g de chlorure de N-(phénacylthio-5 dithiole-1,2 ylidène-3) morpholinium dans 20 cm3 d'éthanol au reflux, on ajoute une solution de 0,2 cm3 d'acide sulfurique concentré (d = 1,83) dans 5 cm3 d'éthanol. Après refroidissement à une température voisine de 10°C, le produit insoluble formé est séparé par filtration et lavé avec 4 cm3 d'éthanol. Après séchage on obtient 0,8 g de sulfate acide de N-(phénacylthio-5 dithiole-1,2 ylidène-3) morpholinium fondant à 192°C.

EXEMPLE 42 -

A une solution de 3,88 g de benzylamino-5 dithiole-1,2 thione-3 dans 65 cm3 d'acétone au reflux, on ajoute 2,9 g d'iodure de sodium et 3,8 g de chlorure de diméthylcarbamoyle et on maintient

EXEMPLE 39 -

A une solution de 3 g d'anilino-5 dithiole-1,2 thione-3 et de 3,3 g d'iodure de sodium dans 50 cm3 d'acétone au reflux, on ajoute une solution de 3,98 g de bromure de phénacyle dans 10 cm3 d'acétone en 5 minutes et on maintient le reflux sous agitation pendant 1 heure. Après refroidissement à une température voisine de 20°C, le produit insoluble formé est séparé par filtration et lavé successivement avec 2 fois 10 cm3 d'acétone, 3 fois 10 cm3 d'eau distillée puis 2 fois 10 cm3 d'éther éthylique.

Par recristallisation du produit ainsi obtenu dans 600 cm3 d'un mélange 50/50 d'eau distillée et d'éthanol, on obtient 3,5 g d'iodhydrate de phénacylthio-5 phénylimino-3 dithiole-1,2 fondant à 180°C.

On peut préparer la base correspondante de la manière suivante : le sel obtenu précédemment est mis en suspension dans 32 cm3 d'eau distillée. On ajoute alors, sous agitation, 0,84 g de bicarbonate de sodium et 50 cm3 de chlorure de méthylène. Après 5 minutes d'agitation, la phase organique est décantée, lavée par 32 cm3 d'eau distillée, séchée sur sulfate de magnésium en présence de noir décolorant, filtrée et concentrée à sec sous pression réduite (20 mm de mercure ; 2,7 kPa) à 40°C.

Par recristallisation du résidu ainsi obtenu dans 60 cm3 d'éthanol, on obtient 2,03 g de phénacylthio-5 phénylimino-3 dithiole-1,2 fondant à 110°C.

EXEMPLE 40 -

A une solution de 6,46 g d'anilino-5 dithiole-1,2 thione-3 et de 6,6 g d'iodure de sodium dans 135 cm3 d'acétone au reflux, on ajoute 9,15 g d'acétyl-4 chlorocarbonyl-1 pipérazine et on maintient le reflux sous agitation pendant 19 heures. Après refroidissement à une température voisine de 20°C, le produit insoluble formé est séparé par filtration et lavé successivement avec 2 fois 10 cm3 d'acétone, 3 fois 20 cm3 d'eau distillée et 2 fois 10 cm3 d'éther éthylique. On obtient ainsi 9,5 g d'iodhydrate d'(acétyl-4 pipérazinyl) carbonylthio-5 phénylimino-3 dithiole-1,2 fondant à 240°C.

le reflux pendant 45 heures. Après refroidissement à une température voisine de 20°C, le produit insoluble est séparé par filtration et lavé successivement avec 2 fois 15 cm3 d'acétone, 2 fois 20 cm3 d'eau distillée puis 15 cm3 d'acétone. On obtient ainsi 6 g d'iodhydrate de benzylimino-3 diméthylcarbamoylthio-5 dithiole-1,2 fondant à 205°C.

On peut préparer la base correspondante de la manière suivante : le sel obtenu précédemment est mis en suspension dans 59 cm3 d'eau distillée. On ajoute alors, sous agitation, 1,53 g de bicarbonate de sodium et 92 cm3 de chlorure de méthylène. Après 10 minutes d'agitation, la phase organique est lavée par 59 cm3 d'eau distillée, séchée sur sulfate de magnésium en présence de noir décolorant, filtrée et concentrée à sec sous pression réduite (20 mm de mercure ; 2,7 kPa) à 40°C.

Par recristallisation du résidu ainsi obtenu dans 125 cm3 d'éthanol, on obtient 3,8 g de benzylimino-3 diméthylcarbamoylthio-5 dithiole-1,2 fondant à 135°C.

La benzylamino-5 dithiole-1,2 thione-3 peut être préparée par action de 26,4 g de pentasulfure de phosphore sur 17,6 g de benzylamino-3 oxo-3 propionate d'éthyle dans 175 cm3 de dioxanne au reflux pendant 30 minutes. Après refroidissement à une température voisine de 70°C, le mélange réactionnel est hydrolysé par 400 cm3 d'une solution aqueuse d'ammoniaque (d = 0,92) à 10 % sous agitation pendant 30 minutes et extrait par 2 fois 100 cm3 de chlorure de méthylène. Les phases organiques réunies sont lavées par 100 cm3 d'eau distillée, séchées sur sulfate de magnésium en présence de noir décolorant, filtrées et concentrées à sec sous pression réduite (20 mm de mercure ; 2,7 kPa) à 50°C. Le résidu obtenu est dissous dans 100 cm3 de chlorure de méthylène et la solution obtenue est versée sur 320 g de gel de silice contenus dans une colonne de 5 cm de diamètre. On élue avec 4,5 litres de chlorure de méthylène ; l'éluat est éliminé. On élue ensuite avec 2 litres de chlorure de méthylène puis avec 2 litres

d'un mélange chlorure de méthylène-méthanol (99-1 en volumes). Ces éluats sont réunis et concentrés à sec sous pression réduite (20 mm de mercure ; 2,7 kPa) à 40°C. Le résidu ainsi obtenu est repris avec 5 cm3 de chlorure de méthylène et le produit insoluble est séparé par filtration puis lavé avec 2 cm3 de chlorure de méthylène. On obtient ainsi 4 g de benzylamino-5 dithiole-1,2 thione-3 fondant à 130°C.

EXEMPLE 43 -

A une solution de 6,9 g de phénéthylamino-5 dithiole-1,2 thione-3 dans 100 cm3 d'acétone, on ajoute 4,8 g d'iodure de sodium et on porte au reflux. On ajoute alors en 5 minutes une solution de 6,4 g de chlorure de diméthylcarbamoyle dans 10 cm3 d'acétone et on maintient le reflux pendant 45 heures. Après refroidissement à une température voisine de 20°C, le produit insoluble formé est séparé par filtration et lavé successivement avec 2 fois 10 cm3 d'acétone, 2 fois 10 cm3 d'eau distillée puis 10 cm3 d'acétone. On obtient ainsi 7,85 g d'iodhydrate de diméthylcarbamoylthio-5 phénéthylimino-3 dithiole-1,2 fondant à 198°C.

On peut préparer la base correspondante de la manière suivante : le sel obtenu précédemment est mis en suspension dans 75 cm3 d'eau distillée. On ajoute alors, sous agitation, 1,95 g de bicarbonate de sodium et 120 cm3 de chlorure de méthylène. Après 5 minutes d'agitation, la phase organique est lavée par 75 cm3 d'eau distillée, séchée sur sulfate de magnésium en présence de noir décolorant, filtrée et concentrée à sec sous pression réduite (20 mm de mercure ; 2,7 kPa) à 40°C.

Par recristallisation du résidu ainsi obtenu dans 110 cm3 d'éthanol, on obtient 4,8 g de diméthylcarbamoylthio-5 phénéthylimino-3 dithiole-1,2 fondant à 115°C.

EXEMPLE 44 -

A une suspension de 4,06 g de (pyrrolidinyl-1)-5 dithiole-1,2 thione-3 et de 4,9 g d'iodure de sodium dans 105 cm3 d'acétone, on ajoute 6,15 g de bromoacétyl-2 thiophène et on maintient le mélange réactionnel au reflux pendant 1 heure 1/2.

Après refroidissement le produit insoluble est séparé par filtration et lavé successivement avec 3 fois 20 cm3 d'acétone, 3 fois 20 cm3 d'eau ; 3 fois 20 cm3 d'éther éthylique. Par recristallisation du produit ainsi obtenu dans 4000 cm3 d'éthanol, on obtient 4,9 g d'iodure de N-[(thénoyl-2) méthylthio-5 dithiole-1,2 ylidène-3] pyrrolidinium fondant à 197°C.

EXEMPLE 45 -

Une suspension de 8 g de morpholino-5 dithiole-1,2 thione-3, 11,63 g d'α-bromopropiophénone et 9 g d'iodure de sodium dans 200 cm3 d'acétone est portée au reflux pendant 2 heures1/2. Après refroidissement à une température voisine de 20°C, le produit insoluble est séparé par filtration et lavé successivement avec 3 fois 20 cm3 d'acétone, 5 fois 50 cm3 d'eau et 3 fois 20 cm3 d'acétone.

Par recristallisation du produit ainsi obtenu dans un mélange de 150 cm3 d'éthanol et 25 cm3 d'eau, on obtient 9,5 g d'iodure de N-[(α-méthyl phénacylthio)-5 dithiole-1,2 ylidène-3] morpholinium fondant à 163°C.

EXEMPLE 46 -

Une solution de 4,5 g de phénylamino-5 dithiole-1,2 thione-3, 4,5 g d'iodure de sodium et 2,6 g de chloroformiate d'éthyle dans 120 cm3 d'acétone anhydre est agitée pendant 65 heures à une température voisine de 20°C. Le produit insoluble formé est séparé par filtration, lavé successivement avec 2 fois 20 cm3 d'acétone puis 3 fois 30 cm3 d'eau distillée, puis 2 fois 20 cm3 d'acétone et séché sous pression réduite (20 mm de mercure ; 2,7 kPa) à 20°C.

Par recristallisation du produit ainsi obtenu dans 100 cm3 d'éthanol, on obtient 3,6 g d'iodhydrate d'éthoxy-carbonylthio-5 phénylimino-3 dithiole-1,2 fondant à 112-116°C.

EXEMPLE 47 -

A une suspension de 4,06 g de (pyrrolidinyl-1)-5 dithiole-1,2 thione-3 et de 4,9 g d'iodure de sodium dans 70 cm3 d'acétone, on ajoute 4,9 g d'α-bromo γ-butyrolactone et on maintient le mélange réactionnel au reflux pendant 6 heures. Après refroidissement, le produit insoluble est séparé par filtration et lavé successivement avec 3 fois 20 cm3 d'acétone, 3 fois 20 cm3 d'eau puis 3 fois 20 cm3 d'éther éthylique. Par recristallisation du produit ainsi obtenu dans 235 cm3 de méthanol, on obtient 5,6 g d'iodure de N-[(oxo-2 tétrahydrofuryl-3) thio-5 dithiole-1,2 ylidène-3] pyrrolidinium fondant à 180°C.

EXEMPLE 48-

A une solution de 3,85 g de (méthoxy-3 phénylamino)-5 dithiole-1,2 thione-3 et de 2,7 g d'iodure de sodium dans 50 cm3 d'acétone au reflux, on ajoute une solution de 1,77 g de chlorure de diméthylcarbamoyle dans 10 cm3 d'acétone. Le mélange réactionnel est chauffé au reflux pendant 40 heures. Après refroidissement à une température voisine de 20°C, on filtre le produit insoluble formé et on le lave successivement avec 20 cm3 d'acétone, 3 fois 20 cm3 d'eau distillée et 20 cm3 d'acétone. On obtient ainsi 2,9 g d'iodhydrate de diméthylcarbamoylthio-5 (méthoxy-3 phényl) imino-3 dithiole-1,2, fondant à 205°C.

On peut préparer la base correspondante de la manière suivante : le sel obtenu précédemment est mis en suspension dans 50 cm3 d'eau. On ajoute alors, sous agitation, 0,6 g de bicarbonate de sodium puis 50 cm3 de chlorure de méthylène. Après 5 minutes d'agitation, la phase aqueuse est décantée et lavée avec 20 cm3 de chlorure de méthylène. Les phases chlorométhyléniques sont réunies, lavées avec 2 fois 50 cm3 d'eau distillée, séchées sur sulfate de magnésium, filtrées et concentrées à sec sous pression réduite (20 mm de mercure ; 2,7 kPa) à 40°C.

Par recristallisation du résidu ainsi obtenu dans 40 cm3 d'éthanol, on obtient 1,5 g de diméthylcarbamoylthio-5 (méthoxy-3 phényl) imino-3 dithiole-1,2 fondant à 104-105°C.

La (méthoxy-3 phénylamino)-5 dithiole-1,2 thione-3 peut être préparée par action de 80 g de pentasulfure de phosphore sur 56,5 g de (méthoxy-3 phénylamino)-3 oxo-3 propionate d'éthyle dans 550 cm3 de dioxanne au reflux pendant 5 minutes. Après refroidissement à une température voisine de 45°C, le mélange réactionnel est hydrolysé par 4 litres d'une solution aqueuse d'ammoniaque (d = 0,92) à 10 %, sous agitation pendant 15 minutes et extrait successivement avec 5 fois 500 cm3 de chlorure de méthylène. Les phases chlorométhyléniques sont réunies, lavées avec 3 fois 500 cm3 d'eau distillée, séchées sur sulfate de magnésium en présence de noir décolorant, filtrées et concentrées à sec sous pression réduite (20 mm de mercure ; 2,7 kPa) à 80°C. Le résidu est repris par 30 cm3 de chlorure de méthylène et le produit qui cristallise est séparé par filtration et lavé avec 5 cm3 de chlorure de méthylène puis 3 fois 30 cm3 d'éther éthylique. Après séchage, on obtient ainsi 4,1 g de (méthoxy-3 phénylamino)-5 dithiole-1,2 thione-3 fondant à 131-134°C.

Le (méthoxy-3 phénylamino)-3 oxo-3 propionate d'éthyle peut être préparé par action de 61,5 g de m-anisidine sur 75,3 g de chloroformylacétate d'éthyle en présence de 50,5 g de triéthylamine dans 500 cm3 de chlorure de méthylène à une température voisine de 15°C pendant 16 heures. Après hydrolyse par 200 cm3 d'eau distillée, la phase organique est lavée successivement avec 200 cm3 d'eau distillée, 200 cm3 d'une solution aqueuse d'acide chlorhydrique 1N et 2 fois 200 cm3 d'eau distillée, séchée sur sulfate de magnésium en présence de noir décolorant, filtrée et concentrée à sec sous pression réduite (20 mm de mercure ; 2,7 kPa) à 60°C. On obtient ainsi 108,6 g de (méthoxy-3 phénylamino)-3 oxo-3 propionate d'éthyle sous forme d'une huile brun-orangé.

[Rf = 0,2 ; chromatographie sur couche mince de gel de silice ; solvant : chlorure de méthylène].

EXEMPLE 49-

A une suspension de 10 g d'(isopropyl-4 phényl) amino-5 dithiole-1,2 thione-3 et de 6,7 g d'iodure de sodium dans 130 cm3 d'acétone au reflux, on ajoute en 5 minutes une solution de 4,4 g de chlorure de diméthylcarbamoyle dans 20 cm3 d'acétone et on maintient le reflux pendant 18 heures. Après refroidissement à une température voisine de 20°C, le produit insoluble est séparé par filtration et lavé successivement avec 2 fois 20 cm3 d'acétone, 3 fois 20 cm3 d'eau distillée puis 2 fois 20 cm3 d'acétone. On obtient ainsi 4,85 g d'iodhydrate de diméthylcarbamoylthio-5 (isopropyl-4 phényl)imino-3 dithiole-1,2 fondant à 160°C.

On peut préparer la base correspondante de la manière suivante : le sel obtenu précédemment est mis en suspension dans 45 cm3 d'eau distillée. On ajoute alors, sous agitation, 1,1 g de bicarbonate de sodium et 70 cm3 de chlorure de méthylène. Après 10 minutes d'agitation, la phase organique est décantée, lavée avec 45 cm3 d'eau distillée, séchée sur sulfate de magnésium, filtrée et concentrée à sec sous pression réduite (20 mm de mercure ; 2,7 kPa) à 40°C.

Par recristallisation du résidu ainsi obtenu dans 350 cm3 d'hexane, on obtient 2,3 g de diméthylcarbamoylthio-5 (isopropyl-4 phényl) imino-3 dithiole-1,2 fondant à 112°C.

L'(isopropyl-4 phényl)amino-5 dithiole-1,2 thione-3 peut être préparée par action de 166,5 g de pentasulfure de phosphore sur 124,7 g d'(isopropyl-4 phényl)amino-3 oxo-3 propionate d'éthyle dans 1,25 litre de dioxanne au reflux pendant 15 minutes. Après refroidissement à une température voisine de 20°C, le mélange réactionnel est hydrolysé par 6 litres d'une solution aqueuse d'ammoniaque (d = 0,92) à 20 % sous agitation pendant 45 minutes et extrait avec 1,5 litre de chloroforme puis avec 3 fois 500 cm3 de chloroforme. Les phases organiques réunies sont lavées avec 500 cm3 d'eau distillée, séchées sur sulfate de magnésium, filtrées et concentrées à sec sous pression réduite (20 mm de mercure ; 2,7 kPa) à 30°C. Le résidu obtenu est trituré

avec 250 cm3 de chlorure de méthylène et le produit insoluble est séparé par filtration et lavé successivement avec 3 fois 50 cm3 de chlorure de méthylène puis 2 fois 100 cm3 d'oxyde d'isopropyle. On obtient ainsi 38,1 g d'(isopropyl-4 phényl)amino-5 dithiole-1,2 thione-3 fondant à 213°C.

L'(isopropyl-4 phényl)amino-3 oxo-3 propionate d'éthyle peut être préparé par action de 109,4 g d'isopropyl-4 aniline sur 113 g de chloroformylacétate d'éthyle en présence de 75,8 g de triéthylamine dans 1 litre de chlorure de méthylène à une température voisine de 20°C pendant 16 heures. Après hydrolyse avec 500 cm3 d'eau distillée, la phase organique est décantée et lavée successivement avec 500 cm3 d'eau distillée, 500 cm3 d'une solution aqueuse d'acide chlorhydrique 1N et 4 fois 500 cm3 d'eau distillée, séchée sur sulfate de magnésium en présence de noir décolorant, filtrée et concentrée à sec sous pression réduite (20 mm de mercure ; 2,7 kPa) à 40°C. On obtient ainsi 190,4 g d'(isopropyl-4 phényl) amino-3 oxo-3 propionate d'éthyle sous forme d'une huile orangée.
[Rf = 0,6 ; chromatographie sur couche mince de gel de silice ; solvant : chlorure de méthylène et méthanol (97,5-2,5 en volumes)].

EXEMPLE 50-

A une suspension de 5,48 g de morpholino-5 dithiole-1,2 thione-3 dans 110 cm3 d'éthanol, on ajoute 5,53 g de chlorure de méthoxy-3 phénacyle et on porte le mélange réactionnel au reflux pendant 6 heures. Après refroidissement à une température voisine de 20°C, le produit insoluble est séparé par filtration et lavé avec 2 fois 20 cm3 d'éthanol. Par recristallisation du produit ainsi obtenu dans 135 cm3 d'un mélange éthanol-eau (4,4-1 en volumes), on obtient 5,7 g de chlorure de N-[(méthoxy-3 phénacyl)thio-5 dithiole-1,2 ylidène-3] morpholinium fondant à 140°C avec décomposition.

EXEMPLE 51 -

A une suspension de 3,05 g de (pyrrolidinyl-1)-5 dithiole-1,2 thione-3 et de 3,7 g d'iodure de sodium dans 53 cm3 d'acétone, on ajoute 5,15 g de bromure de méthoxy-2 phénacyle et on maintient le mélange réactionnel au reflux pendant 1 heure. Après refroidissement le produit insoluble est séparé par filtration et lavé successivement avec 3 fois 20 cm3 d'acétone, 3 fois 20 cm3 d'eau puis 3 fois 20 cm3 d'éther éthylique. Après recristallisation du produit ainsi obtenu dans 1000 cm3 d'éthanol, on obtient 5,3 g d'iodure de N-[(méthoxy-2 phénacylthio)-5 dithiole-1,2 ylidène-3] pyrrolidinium fondant à 205°C.

EXEMPLE 52 -

A une suspension de 5,1 g de (pyrrolidinyl-1)-5 dithiole-1,2 thione-3 et de 6,2 g d'iodure de sodium dans 90 cm3 d'acétone on ajoute 7 g de chlorure de dihydroxy-3,4 phénacyle et on maintient le mélange réactionnel au reflux pendant 1 heure. Après refroidissement, le produit insoluble est séparé par filtration et lavé successivement avec 3 fois 20 cm3 d'acétone, 3 fois 20 cm3 d'eau puis 3 fois 20 cm3 d'éther éthylique. Par recristallisation du produit ainsi obtenu dans 600 cm3 d'un mélange diméthylformamide-méthanol (20-80 en volumes) puis désolvatation par agitation pendant 30 minutes dans 100 cm3 d'eau, on obtient 6,9 g d'iodure de N-[(dihydroxy-3,4 phénacylthio)-5 dithiole-1,2 ylidène-3] pyrrolidinium hydraté, fondant vers 130°C.

EXEMPLE 53 -

A une solution de 2,03 g de (pyrrolidinyl-1)-5 dithiole-1,2 thione-3 et de 2,45 g d'iodure de sodium dans 35 cm3 d'acétone, on ajoute 2,6 g de chlorure de fluoro-4 phénacyle et on maintient le mélange réactionnel au reflux pendant 2 heures. Après refroidissement le produit insoluble est séparé par filtration et lavé successivement avec 3 fois 5 cm3 d'acétone, 3 fois 5 cm3 d'eau puis 3 fois 5 cm3 d'éther éthylique. Par recristallisation du produit ainsi obtenu dans 235 cm3 de méthanol on obtient 2,75 g d'iodure de N-[(fluoro-4 phénacylthio)-5 dithiole-1,2 ylidène-3] pyrrolidinium fondant à 192°C.

EXEMPLE 54 -

Une suspension de 8 g de morpholino-5 dithiole-1,2 thione-3 et de 7,54 g de chlorure de fluoro-4 phénacyle dans 160 cm3 d'éthanol est portée au reflux pendant 6 heures 30 minutes. Après refroidissement à une température voisine de 20°C, le produit insoluble formé est séparé par filtration et lavé successivement avec 3 fois 30 cm3 d'éthanol et 50 cm3 d'éther éthylique.

Par recristallisation du produit ainsi obtenu dans 220 cm3 d'éthanol, on obtient 12,3 g de chlorure de N-[(fluoro-4 phénacylthio)-5 dithiole-1,2 ylidène-3] morpholinium fondant vers 190°C.

EXEMPLE 55 -

Une suspension de 2,7 g de morpholino-5 dithiole-1,2 thione-3 et de 2,52 g de chlorure d'hydroxy-4 phénacyle dans 54 cm3 d'éthanol est portée au reflux pendant 8 heures. Après refroidissement à une température voisine de 20°C, le produit insoluble est séparé par filtration et lavé avec 2 fois 10 cm3 d'éthanol.

Par recristallisation du produit ainsi obtenu dans 100 cm3 d'éthanol, on obtient 3,1 g de chlorure de N-[(hydroxy-4 phénacylthio)-5 dithiole-1,2 ylidène-3] morpholinium fondant vers 180°C.

EXEMPLE 56 -

Une suspension de 8 g de morpholino-5 dithiole-1,2 thione-3 et de 9,2 g de chlorure de chloro-4 phénacyle dans 160 cm3 d'éthanol est portée au reflux pendant 10 heures. Après refroidissement à une température voisine de 20°C, le produit insoluble est séparé par filtration et lavé successivement avec 3 fois 30 cm3 d'éthanol et 50 cm3 d'éther éthylique.

Par recristallisation du produit ainsi obtenu dans un mélange de 450 cm3 d'éthanol et 50 cm3 d'eau, on obtient 8,85 g de chlorure de N-[(chloro-4 phénacylthio)-5 dithiole-1,2 ylidène-3] morpholinium fondant à 160°C.

EXEMPLE 57 -

A une suspension de 8 g de morpholino-5 dithiole-1,2 thione-3 dans 160 cm3 d'éthanol, on ajoute en une fois 9,8 g de chlorure de dichloro-2,4 phénacyle et on porte la suspension ainsi obtenue au reflux pendant 11 heures. Après refroidissement à une température voisine de 20°C, le produit insoluble est séparé par filtration et lavé successivement avec 3 fois 30 cm3 d'éthanol et 50 cm3 d'éther éthylique.

Le produit ainsi obtenu est mis en suspension dans 150 cm3 de chlorure de méthylène et, après agitation pendant 1 heure 30, l'insoluble est séparé par filtration. Après séchage, on obtient 8,2 g de chlorure de N-[(dichloro-2,4 phénacylthio)-5 dithiole-1,2 ylidène-3] morpholinium fondant à 260-262°C.

EXEMPLE 58 -

Une suspension de 8 g de morpholino-5 dithiole-1,2 thione-3 et 7,41 g de chlorure d'amino-4 phénacyle dans 160 cm3 d'éthanol est portée au reflux pendant 7 heures. Après refroidissement à une température voisine de 20°C, le produit insoluble est séparé par filtration et lavé avec 2 fois 20 cm3 d'éthanol.

Le produit ainsi obtenu est déposé sur 1 kg de gel de silice contenu dans une colonne de 4,5 cm de diamètre. On élue successivement avec 1000 cm3 d'un mélange chlorure de méthylène - méthanol (99/1 en volumes), 500 cm3 d'un mélange chlorure de méthylène - méthanol (98/2 en volumes), 500 cm3 d'un mélange chlorure de méthylène - méthanol (95/5 en volumes) et 1000 cm3 d'un mélange chlorure de méthylène - méthanol (90/10 en volumes) ; les éluats correspondants sont éliminés. On élue ensuite successivement avec 1000 cm3 d'un mélange chlorure de méthylène - méthanol (80/20 en volumes), 1000 cm3 d'un mélange chlorure de méthylène - méthanol (60/40 en volumes), 1000 cm3 d'un mélange chlorure de méthylène - méthanol (50/50 en volumes) et 1000 cm3 d'un mélange chlorure de méthylène - méthanol (25/75 en volumes) ; les éluats correspondants sont réunis et concentrés à sec sous pression réduite (20 mm de mercure ; 2,7 kPa) à 40°C. Le produit ainsi obtenu est

mis en suspension dans 100 cm3 de chlorure de méthylène et, après agitation pendant 15 minutes, l'insoluble est séparé par filtration. Après séchage, on obtient 7 g de chlorure de N-[(amino-4 phénacylthio)-5 dithiole-1,2 ylidène-3] morpholinium fondant à 220°C.

EXEMPLE 59 -

Une suspension de 8 g de morpholino-5 dithiole-1,2 thione-3 et de 8,7 g de chlorure de cyano-4 phénacyle dans 160 cm3 d'éthanol est portée au reflux pendant 6 heures. Après refroidissement à une température voisine de 20°C, le produit insoluble est séparé par filtration et lavé successivement avec 3 fois 30 cm3 d'éthanol, 30 cm3 de sulfure de carbone et 30 cm3 d'éther éthylique.

Par recristallisation du produit ainsi obtenu dans un mélange de 900 cm3 d'éthanol et 100 cm3 d'eau, on obtient 9,8 g de chlorure de N-[(cyano-4 phénacylthio)-5 dithiole-1,2 ylidène-3] morpholinium fondant à 198°C.

EXEMPLE 60 -

Une suspension de 8 g de morpholino-5 dithiole-1,2 thione-3 et de 8,8 g de chlorure de chloro-3 phénacyle dans 160 cm3 d'éthanol est portée au reflux pendant 6 heures. Après refroidissement à une température voisine de 20°C, le produit insoluble est séparé par filtration et lavé successivement avec 3 fois 30 cm3 d'éthanol et 50 cm3 d'éther éthylique.

Par recristallisation du produit ainsi obtenu dans un mélange de 315 cm3 d'éthanol et 35 cm3 d'eau, on obtient 10,4 g d'un produit fondant à 180°C. Ce produit est lavé par deux fois 40 cm3 de sulfure de carbone. Par recristallisation dans un mélange de 315 cm3 d'éthanol et 35 cm3 d'eau, on obtient 8,05 g de chlorure de N-[(chloro-3 phénacylthio)-5 dithiole-1,2 ylidène-3] morpholinium fondant vers 175°C.

EXEMPLE 61 -

Une suspension de 5 g de morpholino-5 dithiole-1,2 thione-3, 7,6 g de chlorure de chloro-2 phénacyle et 5,6 g d'iodure de sodium dans 115 cm3 d'acétone est portée au reflux pendant

30 minutes. Après refroidissement à une température voisine de 20°C, le produit insoluble est séparé par filtration et lavé successivement par 20 cm3 d'acétone, 20 cm3 d'eau et 20 cm3 d'éther éthylique.

Par recristallisation du produit ainsi obtenu dans un mélange de 175 cm3 d'éthanol et 175 cm3 d'eau, on obtient 6,75 g d'iodure de N-[(chloro-2 phénacylthio)-5 dithiole-1,2 ylidène-3] morpholinium fondant à 156°C.

EXEMPLE 62 -

Une suspension de 7,9 g de morpholino-5 dithiole-1,2 thione-3 et de 13,55 g de chlorure de méthyl-4 phénacyle dans 160 cm3 d'éthanol est portée au reflux pendant 67 heures. Le produit insoluble est séparé par filtration à une température voisine de 60°C et le filtrat est évaporé à sec sous pression réduite (20 mm de mercure ; 2,7 kPa). Le résidu ainsi obtenu est dissous dans 100 cm3 de chlorure de méthylène et le produit qui cristallise est séparé par filtration puis remis en suspension dans 100 cm3 de chlorure de méthylène. Après agitation pendant 30 minutes à une température voisine de 20°C, l'insoluble est séparé par filtration. On obtient ainsi 4,6 g de chlorure de N-[(méthyl-4 phénacylthio)-5 dithiole-1,2 ylidène-3] morpholinium fondant à 160-164°C.

EXEMPLE 63 -

A une solution de 2,37 g de diméthylcarbamoylthio-5 phénylimino-3 dithiole-1,2, préparé comme à l'exemple 32, dans 50 cm3 d'acétone au reflux, on ajoute 3,4 g d'iodure de méthyle et on maintient le reflux pendant 3 heures. Après refroidissement à une température voisine de 20°C, on filtre le produit insoluble formé et le lave avec 10 cm3 d'acétone.

Après recristallisation dans 100 cm3 d'acétonitrile, on obtient 2,1 g d'iodure de N-méthyl N-(diméthylcarbamoylthio-5 3H-dithiole-1,2 ylidène-3) phénylammonium fondant à 230°C.

EXEMPLE 64 -

Une suspension de 2,62 g de morpholino-5 dithiole-1,2 thione-3, de 4,12 g de bromure de méthoxy-4 phénacyle et de 3 g d'iodure de sodium dans 60 cm3 d'acétone est portée au reflux pendant 2 heures. Après refroidissement à une température voisine de 20°C, le produit insoluble est séparé par filtration et lavé successivement avec 30 cm3 d'acétone, deux fois 40 cm3 d'eau distillée et deux fois 30 cm3 d'acétone. Le produit ainsi obtenu est mis en suspension dans 110 cm3 de chlorure de méthylène. Après agitation pendant une heure à une température voisine de 20°C, le produit insoluble est séparé par filtration ; on obtient ainsi 5,4 g d'iodure de N-[(méthoxy-4 phénacylthio)-5 dithiole-1,2 ylidène-3] morpholinium fondant à 152-155°C.

La présente invention concerne également les médicaments constitués par un produit de formule générale (I) tel que défini ci-avant, sous forme de sel pharmaceutiquement acceptable ou le cas échéant sous forme libre, à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale ou rectale.

Comme compositions solides pour administration orale peuvent être utilisés des comprimés, pilules, poudres (notamment dans des capsules de gélatine ou des cachets) ou granulés. Dans ces compositions le produit actif selon l'invention est mélangé à un ou plusieurs diluants inertes, tels qu' amidon, cellulose, saccharose, lactose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier

des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

En thérapeutique humaine, les produits selon l'invention sont particulièrement utiles comme cytoprotecteurs dans le traitement des ulcères du système digestif. Les doses dépendent de l'effet recherché et de la durée du traitement ; elles sont généralement comprises entre 7 et 700 mg par jour par voie orale pour un adulte en une ou plusieurs prises.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent des compositions selon l'invention.

EXEMPLE A -

On prépare, selon la technique habituelle, des comprimés dosés à 50 mg de produit actif ayant la composition suivante :

- chlorure de N-(méthoxy-3 phénacylthio-5 dithiole-1,2 ylidène-3) morpholinium .......... 50 mg
- amidon .......... 60 mg
- lactose .......... 50 mg
- stéarate de magnésium .......... 2 mg

EXEMPLE B -

On prépare, selon la technique habituelle, des comprimés dosés à 50 mg de produit actif ayant la composition suivante :

| | |
|---|---|
| - iodure de N-[(fluoro-4 phénacylthio)-5, dithiole-1,2 ylidène-3] pyrrolidinium | .......... 50 mg |
| - amidon | .......... 60 mg |
| - lactose | .......... 50 mg |
| - stéarate de magnésium | .......... 2 mg |

REVENDICATIONS

1 - Un nouveau dérivé du dithiole-1,2 ylidène-3 ammonium caractérisé en ce qu'il répond à la formule :

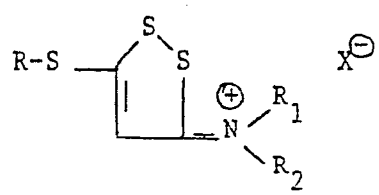

dans laquelle $X^{\ominus}$ représente un anion, R représente un radical alcoyle contenant 1 à 7 atomes de carbone en chaîne droite ou ramifiée [ non substitué ou substitué soit par un radical hydroxy, carboxy, alcoyloxycarbonyle, cyano, dialcoylamino, alcoylcarbonyle, benzoyle dont le cycle phényle est non substitué ou substitué par un ou plusieurs atomes d'halogène ou radicaux choisis parmi alcoyle (éventuellement substitué par un ou plusieurs atomes d'halogène), alcoyloxy, hydroxy, amino, alcoylamino, dialcoylamino, cyano ou nitro, soit par un radical thénoyle dont le cycle thiényle est substitué par un ou plusieurs atomes d'halogène ou radicaux choisis parmi alcoyle, cyano ou nitro, pyridylcarbonyle, carbamoyle, dialcoylcarbamoyle (dont les parties alcoyle peuvent former ensemble avec l'atome d'azote auquel elles sont liées un hétérocycle à 5 ou 6 chaînons contenant éventuellement un autre hétéroatome choisi parmi l'oxygène, le soufre ou un atome d'azote substitué par un radical alcoyle ou alcoylcarbonyle), pyridyle], dialcoylcarbamoyle (dont les parties alcoyle peuvent former ensemble avec l'atome d'azote auquel elles sont liées un hétérocycle à 5 ou 6 chaînons contenant éventuellement un autre hétéroatome choisi parmi l'oxygène, le soufre ou un atome d'azote substitué par un radical alcoyle ou alcoylcarbonyle), alcényle contenant 2 à 4 atomes de carbone, alcynyle contenant 2 à 4 atomes de carbone, alcoyloxycarbonyle ou bien représente un cycle oxo-2 tétrahydrofuryle-3 ou oxo-2 tétrahydropyrannyle-3 et

- soit $R_1$ et $R_2$, identiques ou différents, représentent un radical phényle, cycloalcoyle contenant 3 à 7 atomes de carbone, alcoyle ou phénylalcoyle, ou bien forment ensemble avec l'atome d'azote auquel ils sont liés un hétérocycle à 5 à 7 chaînons pouvant contenir éventuellement un autre hétéroatome choisi parmi l'oxygène, le soufre ou un atome d'azote substitué par un radical alcoyle,

- soit $R_1$ représente un radical phényle non substitué ou substitué par un ou plusieurs atomes d'halogène ou radicaux choisis parmi alcoyle (éventuellement substitué par un ou plusieurs atomes d'halogène), alcoyloxy, hydroxy, amino, alcoylamino, dialcoylamino, cyano ou nitro ou bien représente un radical cycloalcoyle contenant 3 à 7 atomes de carbone, alcoyle ou phénylalcoyle et $R_2$ représente un atome d'hydrogène, ainsi que les bases correspondantes lorsque $R_2$ représente un atome d'hydrogène.

2 - Un procédé de préparation d'un produit selon la revendication 1 caractérisé en ce que l'on fait réagir un dérivé de formule générale

$$R-X_1$$

dans laquelle R est défini comme dans la revendication 1 et $X_1$ représente un atome d'halogène ou un reste d'ester réactif sur une dithiole-1,2 thione-3 de formule générale :

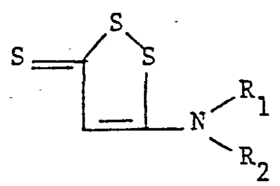

dans laquelle $R_1$ et $R_2$ sont définis comme dans la revendication 1, puis isole le produit obtenu et le transforme éventuellement en un autre sel, ou en base correspondante lorsque $R_2$ représente un atome d'hydrogène.

3 - Un procédé de préparation d'un produit selon la revendication 1 pour lequel $R_1$ représente un radical phényle, cycloalcoyle, alcoyle ou phénylalcoyle et $R_2$ représente un radical cycloalcoyle, alcoyle ou phénylalcoyle, caractérisé en ce que l'on fait réagir un dérivé de formule

$$R'_2X_2$$

dans laquelle $R'_2$ représente un radical cycloalcoyle, alcoyle ou phénylalcoyle et $X_2$ représente un atome d'halogène ou un reste d'ester réactif sur un produit selon la revendication 1 dans la formule duquel $R_1$ représente un radical phényle, cycloalcoyle, alcoyle ou phénylalcoyle et $R_2$ représente un atome d'hydrogène, puis isole le produit obtenu et le transforme éventuellement en un autre sel.

4 - Un médicament caractérisé en ce qu'il contient un produit selon la revendication 1 en association avec un ou plusieurs diluants ou adjuvants compatibles et pharmaceutiquement acceptables.

REVENDICATION

Procédé de préparation d'un nouveau dérivé du dithiole-1,2 ylidène-3 ammonium de formule générale :

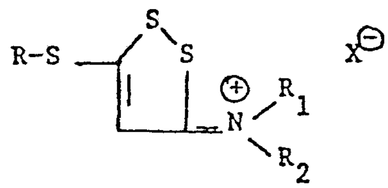

(I)

dans laquelle $X^{\ominus}$ représente un anion, R représente un radical alcoyle contenant 1 à 7 atomes de carbone en chaîne droite ou ramifiée [ non substitué ou substitué soit par un radical hydroxy, carboxy, alcoyloxycarbonyle, cyano, dialcoylamino, alcoylcarbonyle, benzoyle dont le cycle est non substitué ou substitué par un ou plusieurs atomes d'halogène ou radicaux choisis parmi alcoyle (éventuellement substitué par un ou plusieurs atomes d'halogène), alcoyloxy, hydroxy, amino, alcoylamino, dialcoylamino, cyano ou nitro, soit par un radical thénoyle dont le cycle thiényle est substitué par un ou plusieurs atomes d'halogène ou radicaux choisis parmi alcoyle, cyano ou nitro, pyridylcarbonyle, carbamoyle, dialcoylcarbamoyle (dont les parties alcoyle peuvent former ensemble avec l'atome d'azote auquel elles sont liées un hétérocycle à 5 ou 6 chaînons contenant éventuellement un autre hétéroatome choisi parmi l'oxygène, le soufre ou un atome d'azote substitué par un radical alcoyle ou alcoylcarbonyle), pyridyle], dialcoylcarbamoyle (dont les parties alcoyle peuvent former ensemble avec l'atome d'azote auquel elles sont liées un hétérocycle à 5 ou 6 chaînons contenant éventuellement un autre hétéroatome choisi parmi l'oxygène, le soufre ou un atome d'azote substitué par un radical alcoyle ou alcoylcarbonyle), alcényle contenant 2 à 4 atomes de carbone, alcynyle contenant 2 à 4 atomes de carbone, alcoyloxycarbonyle ou bien représente un cycle oxo-2 tétrahydrofuryle-3 ou oxo-2 tétrahydropyrannyle-3 et

- soit $R_1$ et $R_2$, identiques ou différents, représentent un radical phényle, cycloalcoyle contenant 3 à 7 atomes de carbone, alcoyle ou phénylalcoyle, ou bien forment ensemble avec l'atome d'azote auquel ils sont liés un hétérocycle à 5 à 7 chaînons pouvant contenir éventuellement un autre hétéroatome choisi parmi l'oxygène, le soufre ou un atome d'azote substitué par un radical alcoyle,
- soit $R_1$ représente un radical phényle substitué par un ou plusieurs atomes d'halogène ou radicaux choisis parmi alcoyle (éventuellement substitué par un ou plusieurs atomes d'halogène), alcoyloxy, hydroxy, amino, alcoylamino, dialcoylamino, cyano ou nitro ou bien représente un radical cycloalcoyle contenant 3 à 7 atomes de carbone, alcoyle ou phénylalcoyle et $R_2$ représente un atome d'hydrogène, ainsi que les bases correspondantes lorsque $R_2$ représente un atome d'hydrogène,

caractérisé en ce que l'on fait réagir un dérivé de formule générale :

$$R - X_1 \qquad \text{(II)}$$

dans laquelle R est défini comme précédemment et $X_1$ représente un atome d'halogène ou un reste d'ester réactif sur une dithiole-1,2 thione-3 de formule générale :

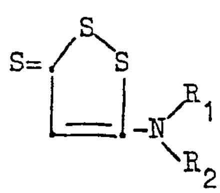

(III)

dans laquelle $R_1$ et $R_2$ sont définis comme précédemment, puis isole le produit obtenu et le transforme éventuellement en un autre sel, ou en base correspondante lorsque $R_2$ représente un atome d'hydrogène, ou en ce que, pour la préparation d'un produit de formule générale (I) dans laquelle $R_1$ représente un radical phényle, cycloalcoyle, alcoyle ou phénylalcoyle et $R_2$ représente un radical cycloalcoyle, alcoyle ou phénylalcoyle, l'on fait réagir un dérivé de formule :

$$R'_2X_2 \qquad \text{(VI)}$$

dans laquelle $R'_2$ représente un radical cycloalcoyle, alcoyle ou phénylalcoyle et $X_2$ représente un atome d'halogène ou un reste d'ester réactif sur un produit de formule générale (I) dans laquelle $R_1$ représente un radical phényle, cycloalcoyle, alcoyle ou phénylalcoyle et $R_2$ représente un atome d'hydrogène, puis isole le produit obtenu et le transforme éventuellement en un autre sel.

Office européen des brevets

# RAPPORT DE RECHERCHE EUROPEENNE

0072743
Numéro de la demande

EP 82 40 1502

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | CHEMICAL ABSTRACTS, vol.75, no.6, 9 août 1971, page 86, résumé no.37870h, Columbus, Ohio (US) H. HARTMANN et al.: "Cationic dyes and their intermediates. V. Simple synthesis of 1,2-dithiolium salts" & J. Prakt.Chem. 1970, 312(6), 1197-1200 * résumé en entier * | 1 | C 07 D 339/04<br>C 07 D 409/02<br>A 61 K 31/385 |
| A | US-A-3 790 677 (J. BADER et al.) * colonnes 1,2 * | 1 | |
| A | FR-A-2 300 793 (OROGIL) * pages 1-3 * | 1 | DOMAINES TECHNIQUES RECHERCHES (Int. Cl. 3) |
| D,A | BULLETIN DE LA SOCIETE CHIMIQUE DE FRANCE, vol.72, no.1, janvier 1972, (FR) G. CAILLAUD et al.: "Composées sulfurés hétérocycliques. LVII. Préparation de bromures d'acylméthylthio-3 dithiole-1,2 ylium", pages 147-161 * pages 148-151 * | 1 | C 07 D 339/00<br>C 07 D 409/00 |

Le présent rapport de recherche a été établi pour toutes les revendications

| Lieu de la recherche | Date d achèvement de la recherche | Examinateur |
|---|---|---|
| LA HAYE | 05-11-1982 | FRANCOIS J.C.L. |

CATEGORIE DES DOCUMENTS CITES

X : particulièrement pertinent à lui seul
Y : particulièrement pertinent en combinaison avec un autre document de la même categorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82